# EUROPEAN PATENT APPLICATION

(11) **EP 3 896 077 A1**
(43) Date of publication of application: **20.10.2021**
(21) Application number: 20169787.7
(22) Date of filing: 16.04.2020
(51) Int. Cl.: C07K 14/005

(54) **INFLUENZA VIRUS-LIKE PARTICLES (VLPS)**

(71) Applicant: Österreichische Agentur für Gesundheit und Ernährungssicherheit GmbH, 1220 Wien (AT)
(72) Inventor: The designation of the inventor has not yet been filed
(74) Representative: SONN Patentanwälte OG

(57) **Abstract**

Disclosed are influenza
virus-like particles (VLPs), wherein the VLPs comprise:
- hemagglutinin (HA) protein and neuraminidase (NA) protein on the surface of the VLPs,
- a nucleoprotein (NP) ribonucleoprotein complex,
wherein the VLPs do not contain a ribonucleoprotein complex of at least one of PB1, PB2, and NS2.

## Description

The present invention discloses influenza virus-like particles (VLPs) and vaccines comprising such VLPs.

A viral infection or vaccination is first recognized by the host's innate immune system. Innate immune cells identify the virus or vaccine as foreign, process it in order to present it to the adaptive immune system and thereby activate it. Depending on the nature of the vaccine, the protection initiated by the adaptive immune system is based more on antibodies (humoral immune response) or on cells that kill newly infected cells quickly and efficiently, making it impossible for the virus to spread (cellular immune response).

Current vaccination strategies against influenza A virus (IAV) are based on the administration of combinations of 3 to 4 inactivated IAV strains or attenuated live viruses (LAIV). Vaccinations with inactivated IAV strains primarily elicit a humoral immune response that only provides protection for about 6 months. Furthermore, this vaccination is only effective against those strains that were used in the manufacture of the vaccine. Moreover, there are concerns over attenuated vaccines about bio safety.

Patients who have survived an IAV infection have broader and more ongoing protection against a new IAV infection, compared to merely vaccinated patients. This protection is provided by the cellular immune response, which is not caused by vaccination with inactivated strains.

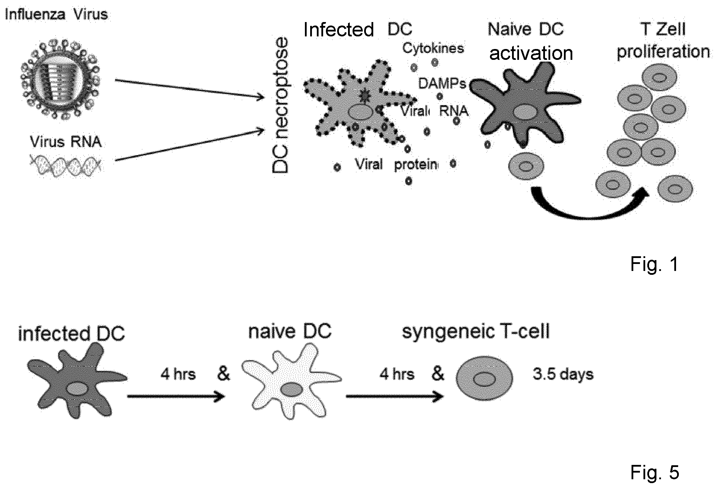

Hartmann et.al. (Nat. Commun. 8 (2017), 1916-1918) discovered that infection with the influenza A virus (IAV) in human dendritic cells (DC) causes inflammatory cell death. The host sensor protein ZBP1/DAI recognizes the viral RNA when it enters the cell and triggers the release of danger signals in the microenvironment. This response induced by viral RNA leads to local inflammation and a more efficient, long-lasting immune response. This suggest that the cellular immune response is activated by the detection of viral RNA newly expressed in the host cell. This in turn means that vaccination with newly expressing viral RNA is necessary for longterm and broader protection (Fig. 1).

Since vaccines based on attenuated viruses have a viral RNA expressing polymerase that is not effective at body temperature8, vaccination in the host does not express the necessary new viral RNA, which triggers the microinflammation required for cellular immunity. For these reasons, the current vaccine strategies have no lasting immune protection against IAV.

Influenza virus causes and 250,000-500,000 deaths worldwide annually; a global pandemic could kill millions. Vaccination is the most cost-effective public health measure to prevent disease and mortality caused by influenza virus infection.

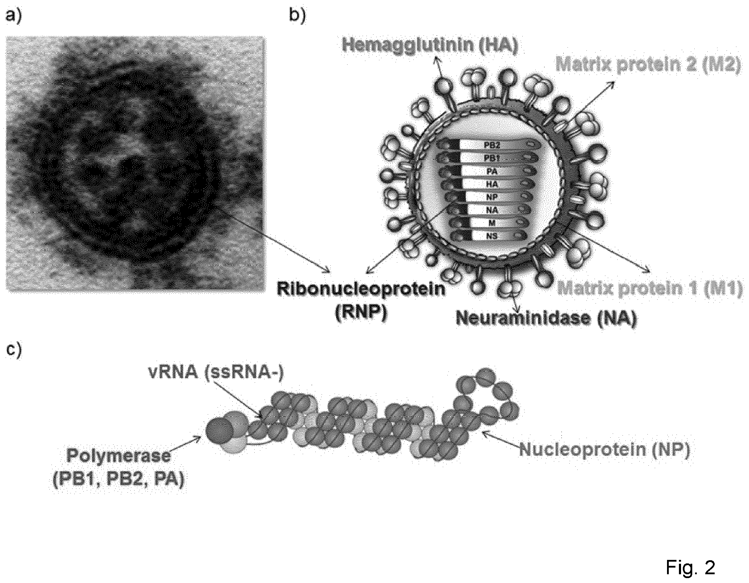

The influenza virion is an enveloped virus whose lipid bilayer comes from the plasma membrane of a host cell. Two different types of glycoprotein spikes are embedded in the shell. Approximately 80 percent of the spikes are hemagglutinin (HA), a trimeric protein that acts when the virus attaches to a host cell. The remaining approximately 20 percent of the glycoprotein spikes consist of neuraminidase (NA), which is believed to primarily facilitate the release of newly produced virus particles from the host cell. There is a matrix protein lining on the inside of the envelope, which surrounds an influenza virion. Inside the envelope is the influenza genome, which is divided into eight single-stranded RNA segments. The RNA is packaged with nucleoprotein (NP) forming a helical ribonucleoprotein complex, containing three polymerase peptides for each RNA segment. Most segments encode a single protein, but some describe several, such as the M segment, which encodes the matrix protein M1 and the ion channel protein M2 (Fig. 2).

Influenza VLPs are potential candidates for inclusion in immunogenic compositions. VLPs closely resemble mature virions, but they do not contain viral genomic material (see e.g. most recently: WO 2020/00101 A1 ([0017], [00254]); WO 2020/014656 A1 (p. 23)). In fact, VLPs are - per definition in the prior art - empty virus envelopes. Therefore, VLPs are non-replicative in nature, which make them safe for administration as a vaccine. In addition, VLPs can be engineered to express viral glycoproteins on the surface of the VLP, which is their most native physiological configuration. Moreover, since VLPs resemble intact virions and are multivalent particulate structures, VLPs may be more effective in inducing neutralizing antibodies to the glycoprotein than soluble envelope protein antigens.

It is an object of the present invention to provide effective and safer vaccination, especially vaccination against influenza virus.

This object of the present invention is solved by the RNA expressing VLPs (RNA-VLPs) according to the present invention.

Therefore, the present invention provides influenza virus-like particles (VLPs), wherein the VLPs comprise:
- hemagglutinin (HA) protein and neuraminidase (NA) protein on the surface of the VLPs,
- a nucleoprotein (NP) ribonucleoprotein complex,
wherein the VLPs do not contain a ribonucleoprotein complex of at least one of PB1, PB2, NS1 and NS2.

The present invention provides RNA-VLPs which are used in a vaccine that appears like a real viral infection to the immune system but cannot multiply. For this purpose, virus-like particles (VLPs) that are externally identical to functional virions were used. Inside the VLP at least one (i.e. NP), preferably two (i.e. NP and M), functional IAV-ribonucleoprotein complexes (RNP) are inserted, which trigger an inflammatory cell death in the host cells like a real virus causes in the host cell by viral RNA expression. In short: after vaccination of the patient, these RNA-VLPs, due to their similarity, enter the cell on the same route as a functional IAV virion and discharge their two RNPs into the cell nucleus. After entry, the viral polymerases which are part of the RNP begin to copy viral RNA, which is then the trigger for the above-described inflammatory cell death (necroptosis). Necroptosis literally causes the host cell to burst, which has several advantages for the immune system: The internal ingredients such as nuclear DNA and proteins are a danger signal that attracts and activates uninvolved cells of the innate immune system; viral proteins and RNA are rendered non-functional by the cell death of infected cells; those non-functional viral parts now serve as pathogen associated molecular patterns (PAMPs), which are taken up by the now activated cells of the innate immune system and, after processing, are presented to the cells of the adaptive immune system. The activation of the innate immune system by the necroptosis of the infected cells induced by the RNA-VLPs is similar to the immune response when infected with a real dangerous IAV virus and is responsible for the activation of a cell-mediated immune response. Vaccinations with classic vaccines do not activate necroptosis due to the lack of RNA expression and therefore induce mainly a humoral immune response. Furthermore, RNA-VLPs are biologically safer than pseudotyped vaccines, which are in short IAV viruses with a non-functional NA protein (Powell et al., J. Virol. 86 (2012), 13397-13406), because they do not replicate segments expressing viral RNA polymerases.

Accordingly, the VLPs according to the present invention trigger an inflammatory cell death (necroptosis) upon entry into a cell but do not trigger apoptosis of the cell.

The role of programmed cell death in influenza virus propagation via caspase-dependent apoptosis has been most extensively examined (see e.g. Harold et al., J. Leukoc. Biol. 92 (2012), 75-82 for review). On the other hand, programmed necrosis (or necroptosis) is a more immunogenic host cell death mechanism (see e.g. Sridharan et al., Trends Microbiol. 22 (2014), 199-207 for review). Unlike apoptosis, necroptosis results in the release of danger-associated molecular patterns (DAMPs), and thus is associated with inflammation and immune cell activation. Necroptosis is receptor-interacting protein kinase (RIPK)- and mixed lineage kinase domain-like pseudokinase (MLKL)-dependent. Depending on the host/pathogen context, necroptosis can either be involved in host response to infection or be exploited by the pathogen for further dissemination.

The RNA-VLPs according to the present invention are an improvement of the existing VLP technology (disclosed e.g. Kang et al., PloS one 14 (2019), e0216871; 10.1371/journal.pone.0216871; Haynes, Exp. Rev. Vacc. 8 (2009), 435-445; Schotsaert et al., Sci. Rep. 6 (2016), 24402; 10.1038/srep24402; Mohan, T. et al., Sci. Rep. 7 (2017), 40226; 10.1038/srep40226), since RNA-VLPs for the immune system simulate infection with a living virus through RNA expression.

The RNA-VLPs according to the present invention can be produced using "reverse genetics" technology (Perez et al., Meth. Mol. Biol. 1602 (2017), 251-273; 10.1007/978-1-4939-6964-7_16). For this purpose, the individual influenza segments are isolated from a field virus, transcribed from RNA into DNA and cloned into DNA plasmids. The plasmids containing the DNA of each IAV segment are then transfected into a cell line. In the transfected cells the plasmids produce either viral copy RNA, and/or viral messenger RNA for the production of viral proteins. The placement of promoters on the plasmids determines whether viral RNA, viral proteins or both are expressed after transfection of the plasmids into a cell culture. This technology has long been used to produce chimeric viruses.

In general, a VLP is known in the present field of technology as particles made up of one of more viral structural proteins, but lacking the viral genome i.a. encoding these viral structural proteins of the VLP. Because VLPs lack a viral genome, they are not able to multiply in cells to generate infectious particles and yield safer and potentially more-economical vaccines and vaccine products. In addition, VLPs can often be produced by heterologous expression and can be easily purified. Most VLPs disclosed in the prior art comprise at least a viral core protein that drives budding and release of particles from a host cell. Influenza VLPs can be produced by transfection of host cells with plasmids encoding the HA, NA and M proteins. After incubation of the transfected cells for an appropriate time to allow for protein expression (such as for approximately 72 hours), VLPs can be isolated from cell culture supernatants. By way of example, a protocol for purifying or isolating influenza VLPs from cell supernatants involves low speed centrifugation (to remove cell debris), vacuum filtration and ultracentrifugation of the VLPs through 20% glycerol. A virus-like particle may also include a subviral particle (SVP), which is typically smaller in size than a virus and constitutes a particle without a virus capsid or genome.

The present VLPs - although containing certain viral nucleic acids - are clearly different from rescued viruses known in the art (e.g. Fodor et al., J. Virol. 73 (1999), 9679-9682; Hoffman et al., Arch. Virol. 146 (2001), 2275-2289; WO 01/04333 A1; WO 2007/016598 A2) due to the lack of an ability to be reproduced in a cell to obtain infectious viral particles.

The VLPs according to the present invention are free of a nucleic acid set-up which would enable the creation of infectious particles in a host cell. This is why - by definition - the VLPs according to the present invention lack at least one of the ribonucleoprotein complexes which are indispensable for viral replication, i.e. PB1, PB2, and NS2 (NS1 has been shown to be dispensable for viral replication in substrates deficient in the IFN pathway (Garcia-Sastre et al., Virol. 252 (1998), 324-330); the characterization of the biological and molecular properties of a recombinant influenza delNS1 virus suggested that NS1 is a virally encoded inhibitor of the IFN-mediated antiviral responses). Preferably, the VLPs according to the present invention lack even more than just one ribonucleoprotein complex of PB1, PB2, and NS2, e.g. two, or all three; it is also preferred that the VLPs according to the present invention lack an NS1 ribonucleoprotein complex. According to the most preferred embodiment of the present invention, the VLPs according to the present invention only contains an NP ribonucleoprotein complex, preferably with an M ribonucleoprotein complex, as the only ribonucleoprotein complexes.

In fact, the VLPs according to the present invention are - due to the mandatory lack of indispensable genetic material for reproducibility within a host cell (i.e. PB1, PB2, and/or NS2) - non-infectious, since they are only able to bind to and enter a cell but not able to be propagated by any host cell to produce infectious particles which then are able to enter further cells.

In contrast to VLPs in the prior art, the VLPs according to the present invention contain viral genetic material (a NP ribonucleoprotein complex; preferably also an M ribonucleoprotein complex) but are - as the prior art VLPs - non-replicating, non-infectious viral capsid structures of influenza. Accordingly, the VLPs according to the present invention are able to enter a cell but no reproduction of the virus/VLP in the cell.

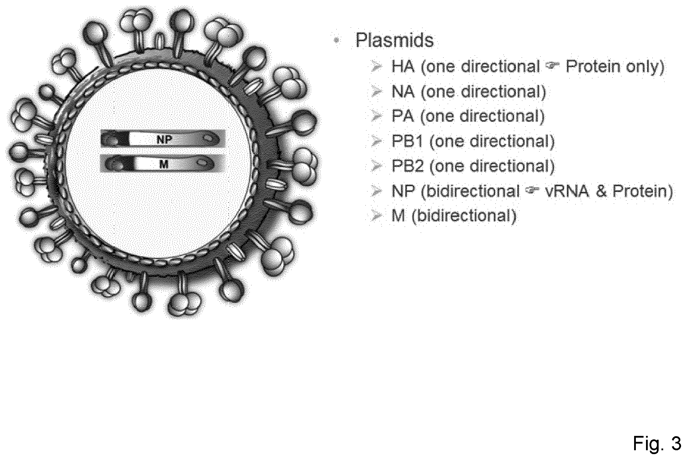

As a minimum the IAV surface proteins hemagglutinin (HA) and neuraminidase (NA) are required, for the construction of the VLPs envelope. The M protein is added, for greater stability and also to make the VLP externally identical to a true virus. The RNP of the NP segment will serve as a source for the viral RNA. NP has the advantages that it is expressed in relatively high numbers early after infection and it also has no known immune antagonistic function. The addition of the M segment as an RNP enables RNA-VLPs to be used as vectors in vaccination against pathogens other than influenza, since foreign sequences of other pathogens can also be added to the M segment without severely restricting its functionality (Fig. 3).

Accordingly, the VLPs according to the present invention preferably exhibit hemagglutinin activity and/or neuraminidase activity. As used herein, the term "hemagglutinin activity" refers to the ability of HA-containing VLPs, or portions thereof to bind and agglutinate red blood cells (erythrocytes). As used herein, the term "neuraminidase activity" refers to the enzymatic activity of NA-containing VLPs, or portions thereof to cleave sialic acid residues from substrates including proteins such as fetuin.

Influenza virus hemagglutinin HA is a viral surface glycoprotein that generally comprises approximately 560 amino acids (e.g., 566 amino acids) and represents 25% of the total virus protein. HA is a protein antigen that is highly useful as an immunogen and is responsible for adhesion of the viral particle to, and its penetration into, a host cell, particularly, in the respiratory epithelium, in the early stages of infection (or entry into a cell (for the VLPs according to the present invention). Eighteen different HA subtypes have been identified in influenza viruses (H1-H18). Influenza virus neuraminidase (NA) is a second membrane glycoprotein of influenza viruses. The presence of viral NA has been shown to be important for generating a multi-faceted protective immune response against an infecting virus. For most influenza A viruses, NA is 413 amino acid in length, and is encoded by a gene of 1413 nucleotides. Nine different NA subtypes have been identified in influenza viruses (NI, N2, N3, N4, N5, N6, N7, N8 and N9), all of which have been found among wild birds. NA is involved in the destruction of the cellular receptor for the viral HA by cleaving terminal neuraminic acid (also called sialic acid) residues from carbohydrate moieties on the surfaces of infected cells. NA also cleaves sialic acid residues from viral proteins, preventing aggregation of viruses. Using this mechanism, it is hypothesized that NA facilitates the release of viral progeny by preventing newly formed viral particles from accumulating along the cell membrane, as well as by promoting transportation of the virus through the mucus present on the mucosal surface. NA is an important antigenic determinant that is subject to antigenic variation.

Sequences of HA and NA proteins to be used according to the present invention are e.g. available from the Influenza Research Database (IRD). The IRD contains avian and non-human mammalian influenza surveillance data, human clinical data associated with virus extracts, phenotypic characteristics of viruses isolated from extracts, and all genomic and proteomic data available in public repositories for influenza viruses. IRD links host surveillance and clinical data to sequence and phenotypic data for all well characterized influenza virus strains as well as data obtained from public data sources for well characterized virus strains will be supplemented with IRD generated data. As of 28 March 2020, the IRD contains 95,032 entries for HA proteins and 82,794 entries for NA proteins.

According to a preferred embodiment, the HA protein in the VLPs according to the present invention is selected from the group of HA proteins disclosed in the IRD, preferably a seasonal, non-pandemic HA. In the IRD, this can be selected by e.g. excluding 2009 pH1N1 sequences from the "Clade Classification" in a search for HA proteins.

Preferably, the NA protein in the VLPs according to the present invention is selected from the group of NA proteins disclosed in the IRD, preferably a seasonal, non-pandemic HA].

Of course, also modified/recombinant versions of HA and NA proteins, e.g. as disclosed in WO 2020/000101 A1).

Influenza A virus RNA segment 5 encodes NP (a polypeptide of 498 amino acids in length), which is rich in arginine, glycine and serine residues and has a net positive charge at neutral pH. The majority of the polypeptide has a preponderance of basic amino acids and an overall predicted pi of 9.3, but the C-terminal 30 residues of NP are, with a pi of 3.7, markedly acidic. In influenza B and C viruses, the length of the homologous NP polypeptide is 560 and 565 residues, respectively. Alignment of the predicted amino acid sequences of the NP genes of the three influenza virus types reveals significant similarity among the three proteins, with the type A and B NPs showing the highest degree of conservation. Phylogenetic analysis of virus strains isolated from different hosts reveals that the NP gene is relatively well conserved.

The seventh RNA segment of influenza viruses (the M gene) encodes both, the M1 and the M2 protein is encoded on together with the M1 protein. M1 is a matrix protein that lies just beneath the viral envelope in the form of dimers and interacts with viral ribonucleoprotein (vRNP) complex, forming a bridge between the inner core components and the membrane proteins. vRNPs harbour the determinants for host range. M1 contacts with both viral RNA and NP, promoting the formation of RNP complexes and causing the dissociation of RNP from the nuclear matrix. M1 plays a vital role in assembly by recruiting the viral components to the site of assembly and essential role in the budding process including formation of viral particles. The M1 protein forms a layer under the patches of host cell membrane that are rich with the viral hemagglutinin, neuraminidase and M2 transmembrane proteins, and facilitates budding of the mature viruses. M1 consists of two domains connected by a linker sequence. The N-terminal domain has a multi-helical structure that can be divided into two subdomains. The C-terminal domain also contains alpha-helical structure. M2 is a membrane protein which is inserted into the viral envelope and projects from the surface of the virus as tetramers. The extracellular domain of M2 is recognized by hosts' immune system. Transmembrane domain of M2 has ion channel activity, which involved in uncoating process of the virus in the cell. The M2 protein is a proton-selective viroporin, integral in the viral envelope of the influenza A virus. The channel itself is a homotetramer (consists of four identical M2 units), where the units are helices stabilized by two disulfide bonds, and is activated by low pH. Proton conductance by the M2 protein in influenza A is essential for viral replication. The M gene therefore encodes both matrix and membrane proteins, and has multiple functions (see e.g. Furuse et al., Virol. J. 6 (2009), 67; doi:10.1186/1743-422X-6-67).

A preferred embodiment of the present VLPs further comprises a matrix protein (M) ribonucleoprotein complex.

According to a preferred embodiment, VLPs according to the present invention comprise:
- HA protein and NA protein on the surface of the VLPs,
- a NP ribonucleoprotein complex, and, preferably an M ribonucleoprotein complex,
wherein the VLPs do not contain a ribonucleoprotein complex of at least two of PB1, PB2, NS1 and NS2; preferably wherein the VLPs do not contain a ribonucleoprotein complex of at least three of PB1, PB2, NS1 and NS2; even more preferred wherein the VLPs do not contain a ribonucleoprotein complex of NS1; especially wherein the VLPs do not contain a ribonucleoprotein complex of PB1, PB2, NS1 and NS2.

Preferably, the M ribonucleoprotein complex in the VLPs according to the present invention contains nucleic acids selected from the group of M genes disclosed in the IRD. Moreover, the M protein encoding nucleic acid sequences in the VLPs according to the present invention may be provided as encoding a modified protein, for example a fusion protein comprising or consisting of (an) influenza M protein(s) and an antigen from a non-influenza virus pathogen. According to this preferred embodiment, the VLPs according to the present invention further comprise a genetically modified M ribonucleoprotein complex, preferably wherein the M ribonucleoprotein complex contains viral RNA encoding an antigen of a non-influenza virus pathogen, especially wherein the M ribonucleoprotein complex contains viral RNA encoding a fusion protein containing an M protein and a protein or antigenic protein fragment of a non-influenza virus pathogen.

For example, the non-influenza virus pathogen can be a non-influenza virus, a gram-positive or gram-negative bacterium, a fungus, a protozoan, or a prion, preferably a non-influenza RNA virus, a DNA virus, or a retrovirus, especially wherein the virus is SARS-COV-1, SARS-COV-2, or Dengue virus.

Preferably, the M ribonucleoprotein complex comprises an RNA encoding a fusion protein comprising the (influenza) M protein and a pathogen antigen or an immunogenic portion thereof. Preferably, the pathogen is a viral, bacterial, fungal or protozoan pathogen. In this context particularly preferred are antigens from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus, Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus, Hepatitis C Virus, Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family, Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS-COV-1, SARS-COV-2, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia entero-colitica, Yersinia pestis, and Yersinia pseudotuberculosis.

Suitable examples of such antigens are disclosed e.g. in WO 2015/149944 A2.

Since micro inflammations elicited by the VLPs according to the present invention are also suitable to overcome the immune abating environment of tumours, the VLP technology according to the present invention can also a be used to elicit an immune response against oncogenes or malignant tissue proteins (for example, tumour antigens located at the surface of tumour cells and being specific for the tumour cells (i.e. not present on benign cells)).

According to another aspect, the present invention also refers to a vaccine comprising the VLPs according to the present invention and a pharmaceutically acceptable excipient.

Preferably, the VLPs according to the present invention are provided as a pharmaceutical preparation suitable to be applied to human individuals. Therefore, the VLPs according to the present invention may be combined with pharmaceutically acceptable excipients which are the conventional carriers (vehicles) and excipients that are physiologically and pharmaceutically acceptable for use in human subjects. Such pharmaceutically acceptable vehicles are known to the skilled practitioner in the pertinent art and can be readily found, e.g. in Remington's Pharmaceutical Sciences, by E. W. Martin, Mack Publishing Co., Easton, Pa., in its updated/current editions, which describes compositions and formulations suitable for pharmaceutical delivery of one or more therapeutic compositions, such as one or more influenza vaccines, and additional pharmaceutical agents. In general, the nature of a pharmaceutically acceptable carrier depends on the particular mode of administration being employed. For instance, parenteral formulations usually comprise injectable fluids/liquids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers may include, for example, pharmaceutical grades of mannitol, lactose, starch, or magnesium stearate, which typically stabilize and/or increase the half-life of a composition or drug. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

Suitable pharmaceutical compositions of the VLPs according to the present invention for parenteral administration include, without limitation, sterile aqueous or non-aqueous solutions, suspensions, and emulsions. Nonlimiting examples of non-aqueous solvents include propylene glycol, polyethylene glycol, vegetable oils, such as olive oil and canola oil, and injectable organic esters, such as ethyl oleate. Aqueous carriers include water, alcoholic/aqueous solutions, emulsions or suspensions, including saline and buffered media. Parenteral vehicles include, for example, sodium chloride solution, Ringer's dextrose, dextrose and sodium chloride, lactated Ringer's, or fixed oils. Intravenous vehicles include, for example, fluid and nutrient replenishers, electrolyte replenishers (such as those based on Ringer's dextrose), and the like. Preservatives and other additives may also be present in such compositions and preparations, such as, for example, antimicrobials, antioxidants, chelating agents, colorants, stabilizers, inert gases and the like.

Some of the compositions may potentially be administered as a pharmaceutically acceptable acid- or base-addition salt, formed by reaction with inorganic acids, such as hydrochloric acid, hydrobromic acid, perchloric acid, nitric acid, thiocyanic acid, sulfuric acid, and phosphoric acid, and organic acids, such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, tri-alkyl and aryl amines and substituted ethanolamines.

Provided herein are pharmaceutical compositions which include a therapeutically effective amount of the VLPs according to the present invention, alone, or in combination with a pharmaceutically acceptable carrier. A person skilled in the art is well aware of suitable pharmaceutical formulations suitable for the present purposes, e.g. there are many appropriate examples and suggestions for the pharmaceutical formulations of influenza vaccines which are also applicable for the VLPs according to the present invention. Pharmaceutically acceptable carriers include, but are not limited to, saline, buffered saline, dextrose, water, glycerol, ethanol, and combinations thereof. The carrier and composition can be sterile, and the formulation suits the mode of administration. The composition can also contain minor amounts of wetting or emulsifying agents, or pH buffering agents. The composition is preferably a liquid or aqueous solution, suspension, emulsion, or dispersion. A liquid or aqueous composition can be lyophilized and reconstituted with a solution or buffer prior to use. The composition can be formulated as a suppository, with traditional binders and carriers such as triglycerides. Any of the commonly known pharmaceutical carriers, such as sterile saline solution or sesame oil, can be used. The medium can also contain conventional pharmaceutical adjunct materials such as, for example, pharmaceutically acceptable salts to adjust the osmotic pressure, buffers, preservatives and the like. Other media that can be used in the compositions and administration methods as described are normal saline and sesame oil.

Preferably, the vaccine according to the present invention is designed as an intranasal delivered vaccine, i.e. a vaccine which can be administered to an individual intranasally. Accordingly, this intranasal vaccine formulation is preferably designed as aerosol or nasal spray. As the RNA-VLP according to the present invention causes the necessary micro inflammation itself, no additional adjuvant is necessary. Accordingly, this vaccine may be provided even free of any further adjuvant. This is specifically preferred, if a fast, easy and cheap production method is needed.

According to another aspect, the present invention relates to a pharmaceutical composition comprising the VLPs according to the present invention.

Another aspect of the present invention is a method for producing VLPs according to the present invention, comprising the steps of
- providing unidirectional vectors for HA, NA, PA, PB1 and PB2;
- providing a bidirectional vector for NP,
- providing either a unidirectional or bidirectional vector for M,
- expressing the vectors in a recombinant cell system so as to obtain VLPs according to the present invention.

Although (as stated above) further bidirectional vectors may be used as well (as long this set-up does not lead to a reproductive viral particle, i.e. a particle which is infectious after production in the host cell to which the VLP has been delivered); however, preferably only one (for NP), even more preferred only two (for NP and M), bidirectional vectors are used in the production of the VLPs according to the present invention.

Since the present method is preferably intended to provide compositions for use in human medicine, the present method further comprises the steps of filling the VLPs into a final container and finishing the VLPs to a pharmaceutical composition ready for use for administration to human individuals.

Preferred for production of the VPLs according to the present invention is any cell that supports replication of influenza virus and meets acceptable standards for regulatory approval. For regulatory reasons the VLPs according to the present invention are therefore preferably produced in cells/cell lines which are approved by the regulatory authorities. Accordingly, production in in Madin-Darby Canine Kidney (MDCK) cells, African green monkey kidney cells (Vero) cells, 293 cells, 293T cells, porcine kidney (PK) cells, owl monkey kidney (OMK) cells, Madin-Darby bovine kidney (MDBK) cells, chicken embryo kidney (CEK) cells, chicken embryo fibroblasts, primary chick kidney cells, or cells isolated from the chorioallantoic membrane of embryonated chicken eggs, preferably in MDCK cells or Vero cells, especially in MDCK cells is preferred. MDCK cell culture is specifically preferred, since MDCK cells were already approved by the FDA and EMA as cell lines for vaccine production (e.g. Flucelvax and Optaflu). MDCK cells are a model mammalian cell line used in biomedical research. MDCK cells are used for a wide variety of cell biology studies including cell polarity, cell-cell adhesions, collective cell motility, as well as responses to growth factors. It is one of few cell culture models that is suited for 3D cell culture and multicellular rearrangements known as branching morphogenesis.

The present invention also relates to the use of the present VLPs for medical use, preferably for preventing pathogen-caused diseases and for the treatment and prevention of tumour diseases (i.e. in immune oncology), preferably for the prevention or treatment of emerging viral diseases, especially for preventing influenza. Preferred virus-caused emerging diseases to be prevented and treated with the vaccines according to the present invention are those identified in Graham et al. (Graham et al., Nat. Immunol. 19 (2018), 20-28), namely Paramyxoviridaea, especially Measles virus, mumps virus, Nipah virus; Togaviridaea(-alphaviridae), especially Rubella virus, Chikungunya virus, Western equine encephalitis virus, Eastern equine encephalitis virus, Venezuelan equine encephalitis virus, Mayaro virus, Ross River virus, Barmah Forest virus, O'nyong'nyong virus, Semliki Forest virus, Getah virus, Sindbis virus; Reoviridaea, especially Rotavirus, New rotaviruses, Banna virus, Nelson Bay orthoreoviruses; Orthomyxoviridaea, especially Influenza virus A and B, multiple subtypes of influenza A virus, Dhori virus, Thogoto virus, Bourbon virus; Adenoviridaea, especially Adenovirus 4 and 7, Adenovirus 14 or 81 or other serotypes; Rhabdoviridaea; Rabies virus, vesicular stomatitis virus (VSV); Picornaviridaea, especially Poliovirus 1, 2 and 3, hepatitis A virus, EV71, EV-D68, rhinoviruses, Ljungan virus; Papillomaviridaea, especially HPV 6, 11, 16 and 18, as well as other serotypes; Poxviridaea, especially Variola virus, Monkeypox virus; Hepadnaviridaea, especially Hepatitis B virus; Herpesviridaea, especially Varicella virus, CMV, EBV, HSV-1, HSV-2, HHV-6, HHV-7, HHV-8; Flaviviridaea, especially Yellow fever virus, tick-borne encephalitis (TBE), Japanese encephalitis (JE), Dengue virus, HCV, Zika virus, St. Louis encephalitis virus, West Nile virus, Powassan virus, Omsk hemorrhagic fever virus, Murray Valley encephalitis virus, Rocio encephalitis virus, Kyasanur forest virus, Alkhurma virus, Russian spring and summer encephalitis virus, Central European tick-borne encephalitis virus, Wesselsbron virus, Bussuquara virus, Cacipacore virus, Ilheus virus, Iguape virus, Usutu virus; Hepeviridaea, especially Hepatitis E virus; Pneu-moviridae, especially respiratory syncytial virus (RSV); metap-neumovirus; Filoviridae, especially Ebola virus, Marburg virus, Retroviridae, especially HIV-1, Coronaviridae, especially SARS-COV-1, SARS-COV-2, Middle Eastern respiratory syndrome (MERS); Parvoviridae, especially B19 virus, bocavirus; Caliciviridae, especially Norovirus; Polyomaviridae, especially JC virus, BK virus; Arenaviridae, especially Lassa virus, Machupo virus, Junin virus, Guanarito virus, Chapare virus, Sabia virus, Flexal virus, lymphocytic choriomeningitis virus, Lujo virus; Polyomaviridae, especially SV40, Merkel cell virus; Arteriviridaea, especially Simian hemorrhagic fever virus; Bunyaviridae, especially Hantavirus, Rift Valley virus, Crimean Congo hemorrhagic fever virus, California encephalitis virus, Batai virus, Bhanja virus, Dobrava-Belgrade virus, Erve virus, Puumala virus, Seoul virus, Tahyna virus, severe fever with thrombocytopenia syndrome virus, La Crosse encephalitis virus, Cache Valley virus, Jamestown Canyon virus, snowshoe hare virus, Heartland virus, Oropouche virus; Astroviridae, especially Astrovirus. In the VLPs according to the present invention, viral antigens of these viruses can be introduced in the M gene so as to elicit immune responses against this recombinant antigen upon administration of the VLPs according to the present invention (as vaccines) to a (human) individual.

Methods of treating or preventing a disease or infection, or symptoms thereof, caused by influenza virus (or other (viral) diseases) are provided. The methods comprise administering a therapeutically effective amount of a pharmaceutical composition comprising the VLPs according to the present invention (e.g., a VLP vaccine) to a subject (e.g., a mammal, in particular, a human subject). One embodiment involves a method of treating a subject suffering from, or at risk of or susceptible to disease or infection, or a symptom thereof, caused by the pathogen, especially the influenza virus. The method includes administering to the subject (e.g., a mammalian subject), an amount or a therapeutic amount of a vaccine comprising VLPs, sufficient to treat the disease, infection, or symptoms thereof, caused by the pathogen, especially the influenza virus, under conditions in which the disease, infection, and/or the symptoms thereof are treated.

In an embodiment, the methods herein include administering to the subject (including a human subject identified as in need of such treatment) an effective amount of a vaccine according to the present invention to produce such effect. The treatment methods are suitably administered to subjects, particularly humans, suffering from, having, susceptible to, or at risk of having a disease, disorder, infection, or symptom thereof, namely, caused by the pathogen, especially a flu or influenza. Identifying a subject in need of such treatment can be based on the judgment of the subject or of a health care professional and can be subjective (e.g. opinion) or objective (e.g. measurable by a test or diagnostic method).

In addition, prophylactic methods of preventing or protecting against a disease or infection, or symptoms thereof, caused by the pathogen, especially the influenza virus, are provided. Such methods comprise administering a therapeutically effective amount of a pharmaceutical composition comprising an immunogenic composition according to the present invention (e.g., a VLP vaccine) to a subject (e.g., a mammal such as a human), in particular, prior to infection of the subject or prior to onset of the disease, such as influenza.

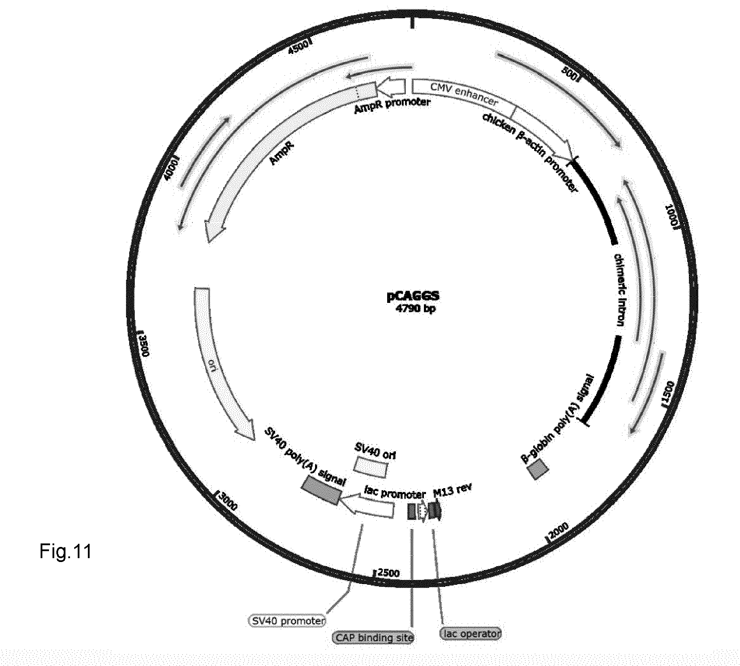

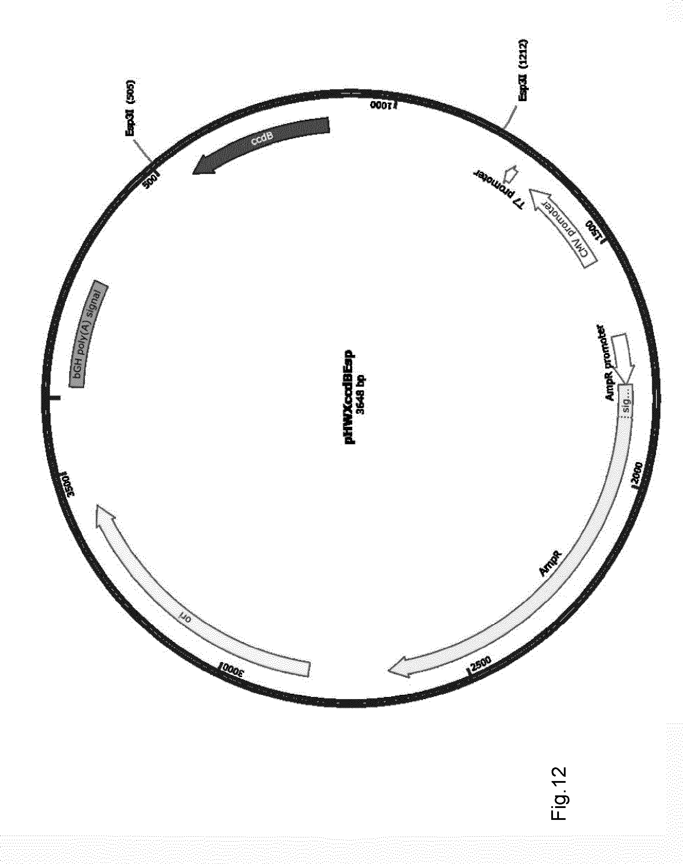

Therefore (and as shown in the following example section), to create the RNA-VLP, the surface molecules HA, NA and the polymerases PA, PB1, PB2 expressed as proteins and the segments NP and M expressed as proteins and RNA are used. HA, NA PA, PB1 and PB2 are used in the construction of the RNA-VLPs in a unidirectional plasmid which only produces viral protein. E.g. PCAGGS (Fig. 11) can used as the unidirectional plasmid for this purpose. After transfection into a cell, this plasmid only expresses messenger RNAs, which are then translated into viral proteins by the cell's own machinery. A bidirectional plasmid, such as pHWXccdB (Fig. 12) can be used for the expression NP and M, into both mRNA as well as viral copy RNA (vRNA). The expression of the negative sense genome RNA, (vRNA), is fostered by a human RNA polymerase I promoter and a terminator sequence, e.g. the mouse terminator sequence on pHWXccdB. In the opposite orientation to the polymerase I unit, a polymerase II transcription cassette (e.g. the chicken β-actin promoter and polyA) encodes the mRNA from the same viral gene. After successful cloning of the segments into the appropriate plasmids, all plasmids are transformed together into a suitable cell line, e.g. the MDCK target line. Self-assembly unites the proteins and RNAs into viral shells with the surface proteins as well as the two RNPs, which contain viral M and NP RNA. The finished RNA-VLPS are then released into the cell culture medium and harvested there.

The invention is further illustrated by the following examples and the figures, yet without being limited thereto.
Fig. 1 shows the principle of triggering cellular immunity by viral RNA. Infection of dendritic cells by influenza or expression of viral RNA triggers necroptosis. The process of necroptosis involves the perforation of cell membranes, which in turn leads to the release of inner-cell constituents, which are perceived as a danger signal by other cells. In addition, the perforation also leaves viral nucleic acids and proteins in the intercellular space and can thus be better processed by the innate immune cells, which were activated by those danger associated molecular patterns (DAMPs), and then more effectively presented to cells of the adaptive immune system such as T cells;
Fig. 2 shows the structure of the influenza (IAV) virion. a) Electron microscope image of an influenza virus particle. b) Graphic representation of the IAV virion: In the lipid bilayer, which originates from a host cell, there are HA and NA spikes and M2 ion channels. The strength of the virion is supported by an envelope made of M1 matrix protein. The genetic information of the virus is divided into 8 segments, which are located inside the virion as a ribonucleoprotein complex. c) Schematic representation of a ribonucleoprotein complex (RNP): The genetic information is wound as a negative single-stranded RNA around a strand of nucleoproteins (NP). Furthermore, the RNPs have their own polymerase complex (PB1, PB2 and PA), which begins to replicate the viral RNA independently after the virion has successfully penetrated the host cell;
Fig. 3 shows the schematic representation of the RNA-VLP and the plasmids necessary for its production: The surface of the VLP is identical to that of an IAV virion (see Fig. 2). Inside the RNA-VLP are two ribonucleoprotein complexes that express viral RNA for NP and M after inoculation de novo. When the RNA-VLP is formed in cell culture, HA, NA, and the polymerases PA, PB1, PB2 are required only as proteins, so the expression of these proteins is accomplished by a plasmid that only produces messenger RNA and the proteins that is translated by the host's cell machinery. Since NP and M are required both as protein and as viral copy RNA (vRNA), a bidirectional plasmid is required which expresses messenger RNA as well as vRNA;

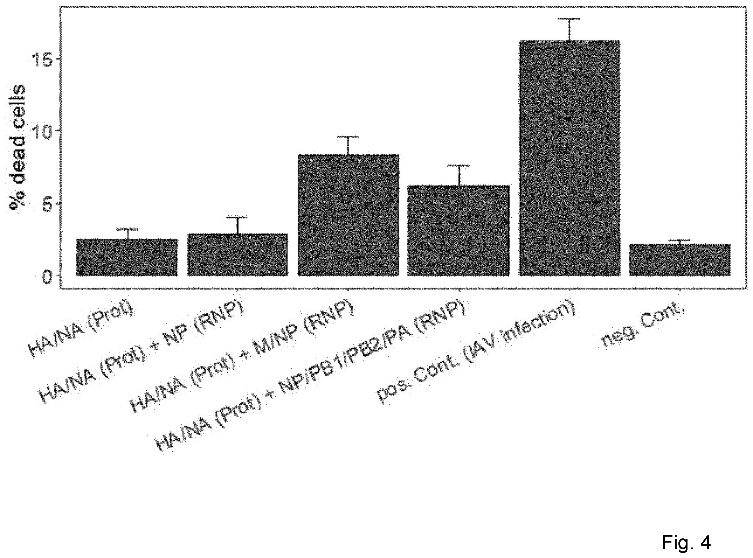

Fig. 4 shows cell death triggered by RNA-VLPs. Human dendritic cells were infected with various constructs of VLPs and, after an eight-hour incubation, analysed for cell death using imaging flow cytometry. An empty VLP (HA / NA (Prot.)) and vehicle (cell culture medium) were used as negative controls. Infection with an influenza virus and a VLP with all three polymerases as additional RNPs served as a positive control;
Fig. 5 shows the experimental set-up to measure the induction of cellular immunity by RNA-VLPs. Human DCs were infected with RNA-VLPs or controls. After a four-hour incubation, DCs from the same donor were added and after another four hours, T cells from another donor were added. The cell death in the infected DCs activates the added DCs, which in turn cause the T cells to proliferate (see Fig. 1);

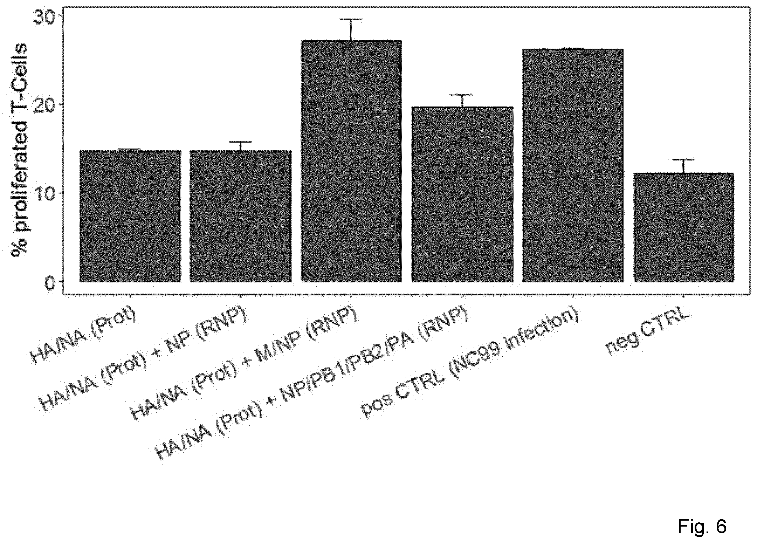

Fig. 6 shows the cellular immunity triggered by RNA-VLPs. Human dendritic cells were infected with various constructs of VLPs and cultured with DCs and foreign T cells (see Fig. 5). T cell proliferation was measured using flow cytometry. An empty VLP (HA / NA (Prot.)) and vehicle infection (cell culture medium) were used as negative controls. Infection with an influenza virus and a VLP containing all three polymerase RNPs served as a positive control;

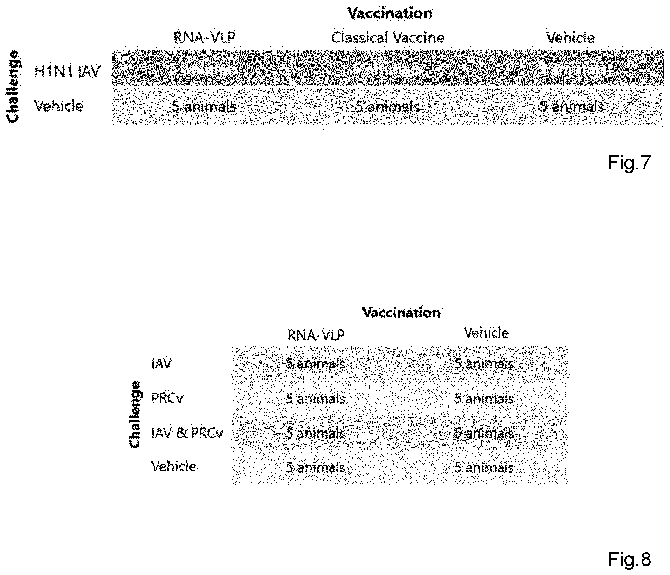

Fig. 7 shows the sample plan IAV challenge study;
Fig. 8 shows the sample plan for the challenge study involving a chimeric vaccine against IAV and PRCv;

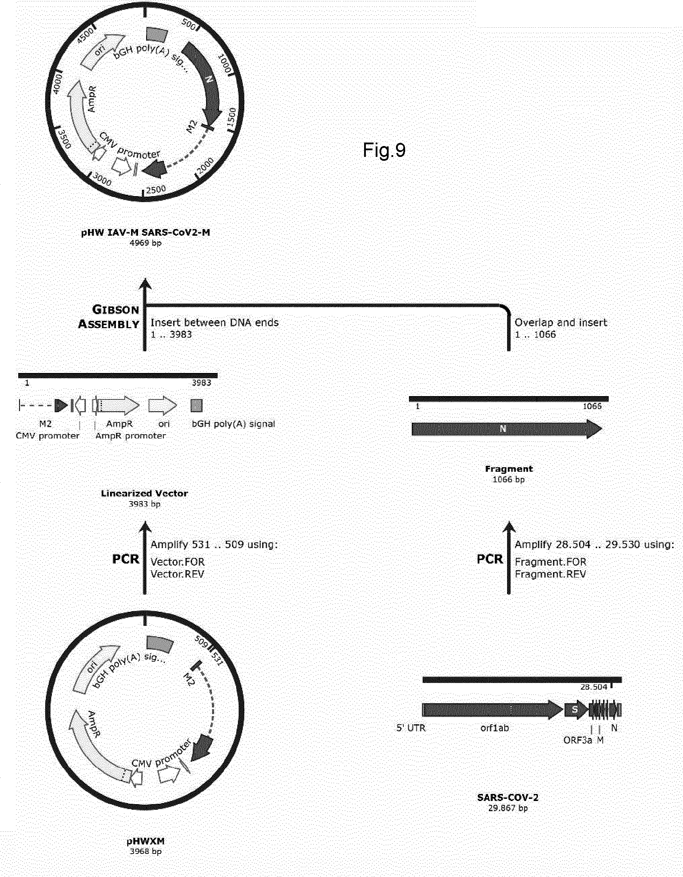

Fig. 9 illustrates the production of the chimeric bidirectional plasmid containing genetic information of the IAV M segment as well as genetic information from the SARS-CoV-2 using Gibson assembly. In short: The phW2000 plasmid containing the IAV M Segment as well as a specific region of the SARS-COV-2 are amplified by using specific primers creating a 20 base pair overlap between the two amplicons. During the assembly process a nuclease chews back DNA from the 5' end allowing the two amplicons to anneal. A DNA polymerase and ligase and fill any gaps and joins the DNA removing any nicks in the DNA. The product of this process is bidirectional plasmid containing the chimeric sequence of IAV M and a SARS-CoV-2 protein;

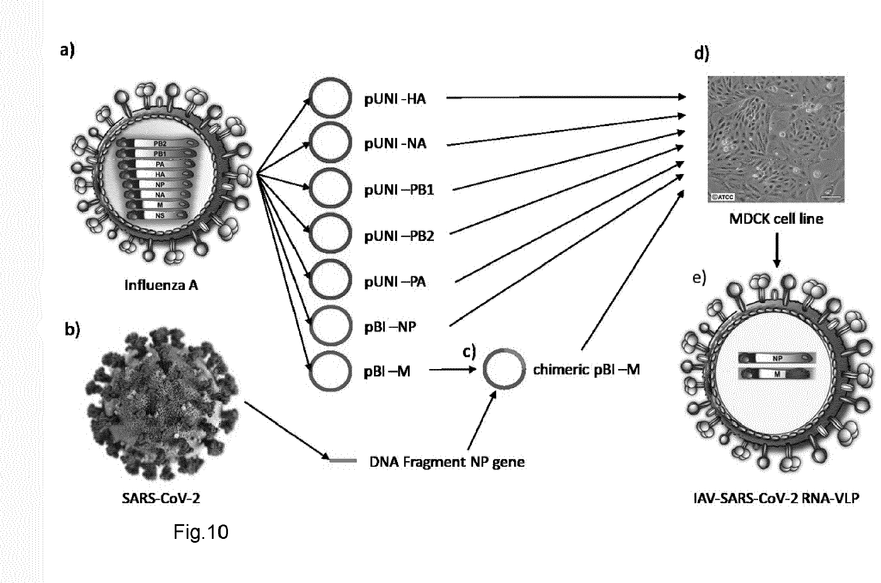

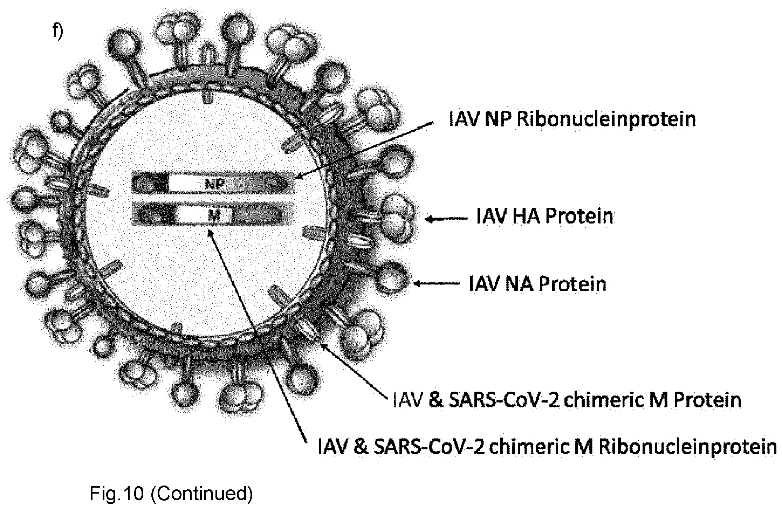

Fig. 10 shows a scheme of chimeric IAV SARS-CoV-2 RNA-VLP production. a) HA, NA, PB1, PB2 and PA segments are rescued in unidirectional (pUNI) plasmids by reverse genetics. NP and M are rescued in bidirectional plasmids (pBI) b) A specific region of the SARS-CoV-2 is reverse transcribed and amplified by PCR. c) A chimeric bidirectional plasmid containing the genetic information of the IAV M segment and a part of SARS-CoV-2 is produced by Gibson assembly (see Fig 9). d) The HA, NA, PB1, PB2, PA, NP and chimeric M plasmid is transformed into a MDCK cell line culture. e) The chimeric IAV-SARS-CoV-2 RNA-VLP arises through self-assembly in the cell line and is harvested in the cell culture supernatant. f) the chimeric IAV-SARS-CoV-2 RNA-VLP in detail;
Fig. 11 shows the plasmid map of pCAGGS;
Fig. 12 shows the plasmid map of pHWX

### EXAMPLES:

### I. In vitro proof of concept for the present invention

To show the functionality of the present invention, the following VLPs with HA and NA surface proteins and different configurations of RNPs were constructed:
1. An empty VLP that has only HA and NA proteins on the surface
2. VLPs with an NP ribonucleoprotein complex
3. VLPs with an NP and M ribonucleoprotein complex. It should be mentioned that due to the bidirectional nature of the pHWX plasmid M, both RNP and surface protein are present in addition to HA and NA
4. VLPs with NP and all polymerases as a ribonucleoprotein complex. This design was designed as a positive control and is not suitable as a vaccine due to biological safety concerns.

For the prototype vaccine, HA and NA from virus strain A/New Caledonia/20/1999 and M, polymerases and NP from virus strain A/ California/4/2009 were used. The individual IAV segments were isolated according to Hoffmann et al. (Arch. Virol. 146 (2001), 2275-2289, 10.1007/s007050170002). First, the vRNA was extracted from virus isolates. The vRNA was rewritten into cDNA using the UNI12 primer and a reverse transcriptase. Segment-specific sequences were then amplified by PCR using segment specific primers and purified by gel electrophoresis (Hoffmann et al., 2001). The individual segments were cloned into their intended plasmids using restriction enzymes and ligases, transformed into E. coli bacteria and plated out on agar plates. Individual colonies were selected to check the cloned insert for completeness and correctness using Sanger sequencing. The plasmids were then transformed together into MDCK cells by lipofectamine. After the transformation, the RNA-VLPs were created by "self-assembly" in the MDCK cells (Nayaket et al., Virus Res. 106 (2004), 147-165; 10.1016/j.vi-rusres.2004.08.012). Next, the RNA-VLPs were harvested in the cell culture medium. In the future, it is planned to use simpler and more effective methods for creating the plasmids and RNA-VLPs, e.g. one pot cloning (Choi et al., Sci. Rep. 9, (2019) 8318; 10.1038/s41598-019-44813-z).

The cell death induction of the VLPs was tested in human dendritic cells as the ability to trigger necroptosis in immune cells is crucial for vaccine effectiveness (Hartmann et al., 2017) . For this purpose, dendritic cells from monocytes, which were obtained from anonymous blood donations, were grown by adding the cytokines GMCSF and IL-4. These dendritic cells were then infected with the various VLP constructs or with the influenza strain NC/99 as a positive control. Eight hours after infection, cell death induction was measured using flow cytometry. While the inclusion of the NP RNP alone into the VLP did not result in a significant increase in cell death rate compared to the uninfected negative control, there was a significant increase in the inclusion of both NP and M RNPs, which was surprisingly higher than the VLPs, were also all polymerases were included as RNPs (Fig 4).

The ability of the VLPs to elicit a cellular immune response was checked by a T-cell dendritic-cell co-cultivation method. For this purpose, human dendritic cells were infected with the VLP constructs or the controls (virus or vehicle). After 4 hours, dendritic cells from the same blood donor were added so that they could be activated by the danger signals from the dead infected cells. This cell mixture was again cultured after 4 hours with T cells from another donor for 2.5 days. These foreign T cells were activated on the one hand by the graft-versus-host reaction and on the other hand by the status of the dendritic cells and thereby driven to proliferation. The proliferation was then measured by flow cytometry using the dilution of a dye which is associated with each cell division (proliferation) (Fig. 5).

Similar to the cell death experiments, the empty VLPs could not trigger an increased T cell proliferation compared to the negative control. This is an indication that vaccination with surface proteins primarily elicits antibody-mediated immunity. The VLPs with M and NP RNPs, on the other hand, were able to produce at least a similarly high T cell proliferation as an infection with a real influenza virus (positive control). Interestingly, the M/NP RNP combination was even more effective than the combination of NP and all three polymerases. The VLP construct containing only the NP RNP was able to cause a slight increase in T cell proliferation. These data show that a VLP consisting of a surface including HA, NA and M proteins filled with NP and M RNPs is a good trigger of cellular anti-viral immunity (Fig. 6).

In summary, the present invention consists of combining the technology of the VLPs and complexes expressing viral RNA and the resulting vaccine looking like an infectious particle for the immune system. After being absorbed into an organism, these RNA-VLPs behave like an infectious IAV virion by penetrating the cell nucleus and beginning to express viral RNA through the polymerases they have brought with them. This newly synthesized RNA then triggers an inflammatory signal cascade that boosts cellular immunity. Exactly this step is not used in current vaccination strategies. We have already proven that this innovation creates the cellular immunity necessary for long-lasting protection in the cell culture system.

### II. In vivo proof of concept for the present invention

In vivo proof of concept is provided by two experiments. The first experiment focuses on testing the effect of RNA-VLP as a sole vaccination against porcine influenza. In a second experiment an RNA-VLP vaccine is constructed with broader activity against influenza and a porcine corona virus, which causes pathologies in pigs comparable to those observed in humans when infected with the current SARS-CoV-2.

### II.1. Proof of concept of vaccination against IAV with RNA-VLPs in pigs.

An RNA-VLP with segments from a recently isolated porcine H1N1 IAV isolate is constructed as described in the sections above. The efficacy of the RNA-VLP as a vaccine against influenza is tested in a challenge experiment where the usefulness of the RNA-VLP is compared with a conventional veterinary anti influenza vaccine. 30 animals are grouped in three groups of 10: (i) animals getting the RNA-VLPs plus vehicle, (ii) animals taking the conventional vaccine, and vehicle and (iii) vehicle group. After vaccination 5 of the ten animals are challenged with an IAV infection (Fig. 7). Clinical signs, laboratory measurements (such as viral load, and cytokine levels), and cell immunological measurements serve as readouts for vaccine efficacy.

### II.2 Proof of concept of combinatorial vaccination against IAV and coronavirus

For this experiment a RNA-VLP is constructed where a part of the nucleoprotein sequence of the porcine respiratory corona virus (PRCv) is added to the M segment RNP. PRCv has a similar structure to SARS-CoV-2 and serves as a model as there is currently no animal model to test SARS-CoV-2 infections. 40 animals are grouped in two groups of 20: (i) animals getting the RNA-VLPs plus vehicle, and (ii) vehicle group. After vaccination groups of five of the twenty animals are challenged with an IAV, PRCv, a combination of IAV and PRCv or vehicle (Fig. 8). Clinical signs, laboratory measurements (such as viral load, and cytokine levels), and cell immunological measurements serve as readouts for vaccine efficacy.

### II.3 Proof of concept of a combinatorial vaccination against IAV and SARS-CoV-2

For this experiment, a RNA-VLP with a chimeric IAV M RNP will be constructed. The chimeric M RNP will contain genetic information of the IAV M segment as well as genetic information fully or partially describing one of the proteins of the SARS-CoV-2. VLP will be constructed as described above. Those VLPs will be used to vaccinate pigs. After vaccination and a possible booster vaccination blood will be drawn from the animals, which will be used in serological and in vitro cell immunological tests. As soon as a valid and useable animal model for SARS-CoV-2 is identified the anti SARS-CoV-2 RNA-VLP will be tested in a in vivo challenge study (Figs. 9 and 10).

### Abbreviations:

- DAI: DNA-dependent activator of IFN-regulatory factors
- DAMPs: danger associated molecular patterns
- DC: dendritic cells
- HA: hemagglutinin
- IAV: Influenza A Virus
- LAIV: attenuated live virus vaccine
- NA: neuraminidase
- NP: nucleoprotein
- PAMPs: pathogen associated molecular patterns
- PRCv: porcine respiratory corona virus
- RNA: Ribonucleic acid
- RNP: ribonucleoprotein complexes
- ZBP1: Z-DNA-binding protein 1

### Sequences:

SEQ ID NO: 1 (pCAGGS):
GTCGACATTGATTATTGACTAGTTATTAATAGTAATCAATTACGGGGTCATTAGTTCATAGCCCATATAT
GGAGTTCCGCGTTACATAACTTACGGTAAATGGCCCGCCTGGCTGACCGCCCAACGACCCCCGCCCATTG
ACGTCAATAATGACGTATGTTCCCATAGTAACGCCAATAGGGACTTTCCATTGACGTCAATGGGTGGACT
ATTTACGGTAAACTGCCCACTTGGCAGTACATCAAGTGTATCATATGCCAAGTACGCCCCCTATTGACGT
CAATGACGGTAAATGGCCCGCCTGGCATTATGCCCAGTACATGACCTTATGGGACTTTCCTACTTGGCAG
TACATCTACGTATTAGTCATCGCTATTACCATGGGTCGAGGTGAGCCCCACGTTCTGCTTCACTCTCCCC
ATCTCCCCCCCCTCCCCACCCCCAATTTTGTATTTATTTATTTTTTAATTATTTTGTGCAGCGATGGGGG
CGGGGGGGGGGGGGGCGCGCGCCAGGCGGGGCGGGGCGGGGCGAGGGGCGGGGCGGGGCGAGGCGGAGAG
GTGCGGCGGCAGCCAATCAGAGCGGCGCGCTCCGAAAGTTTCCTTTTATGGCGAGGCGGCGGCGGCGGCG
GCCCTATAAAAAGCGAAGCGCGCGGCGGGCGGGAGTCGCTGCGTTGCCTTCGCCCCGTGCCCCGCTCCGC
GCCGCCTCGCGCCGCCCGCCCCGGCTCTGACTGACCGCGTTACTCCCACAGGTGAGCGGGCGGGACGGCC
CTTCTCCTCCGGGCTGTAATTAGCGCTTGGTTTAATGACGGCTCGTTTCTTTTCTGTGGCTGCGTGAAAG
CCTTAAAGGGCTCCGGGAGGGCCCTTTGTGCGGGGGGGAGCGGCTCGGGGGGTGCGTGCGTGTGTGTGTG
CGTGGGGAGCGCCGCGTGCGGCCCGCGCTGCCCGGCGGCTGTGAGCGCTGCGGGCGCGGCGCGGGGCTTT
GTGCGCTCCGCGTGTGCGCGAGGGGAGCGCGGCCGGGGGCGGTGCCCCGCGGTGCGGGGGGGCTGCGAGG
GGAACAAAGGCTGCGTGCGGGGTGTGTGCGTGGGGGGGTGAGCAGGGGGTGTGGGCGCGGCGGTCGGGCT
GTAACCCCCCCCTGCACCCCCCTCCCCGAGTTGCTGAGCACGGCCCGGCTTCGGGTGCGGGGCTCCGTGC
GGGGCGTGGCGCGGGGCTCGCCGTGCCGGGCGGGGGGTGGCGGCAGGTGGGGGTGCCGGGCGGGGCGGGG
CCGCCTCGGGCCGGGGAGGGCTCGGGGGAGGGGCGCGGCGGCCCCGGAGCGCCGGCGGCTGTCGAGGCGC
GGCGAGCCGCAGCCATTGCCTTTTATGGTAATCGTGCGAGAGGGCGCAGGGACTTCCTTTGTCCCAAATC
TGGCGGAGCCGAAATCTGGGAGGCGCCGCCGCACCCCCTCTAGCGGGCGCGGGCGAAGCGGTGCGGCGCC
GGCAGGAAGGAAATGGGCGGGGAGGGCCTTCGTGCGTCGCCGCGCCGCCGTCCCCTTCTCCATCTCCAGC
CTCGGGGCTGCCGCAGGGGGACGGCTGCCTTCGGGGGGGACGGGGCAGGGCGGGGTTCGGCTTCTGGCGT
GTGACCGGCGGCTCTAGAGCCTCTGCTAACCATGTTCATGCCTTCTTCTTTTTCCTACAGCTCCTGGGCA
ACGTGCTGGTTGTTGTGCTGTCTCATCATTTTGGCAAAGAATTCCTCGAGGAATTCACTCCTCAGGTGCA
GGCTGCCTATCAGAAGGTGGTGGCTGGTGTGGCCAATGCCCTGGCTCACAAATACCACTGAGATCTTTTT
CCCTCTGCCAAAAATTATGGGGACATCATGAAGCCCCTTGAGCATCTGACTTCTGGCTAATAAAGGAAAT
TTATTTTCATTGCAATAGTGTGTTGGAATTTTTTGTGTCTCTCACTCGGAAGGACATATGGGAGGGCAAA
TCATTTAAAACATCAGAATGAGTATTTGGTTTAGAGTTTGGCAACATATGCCATATGCTGGCTGCCATGA
ACAAAGGTGGCTATAAAGAGGTCATCAGTATATGAAACAGCCCCCTGCTGTCCATTCCTTATTCCATAGA
AAAGCCTTGACTTGAGGTTAGATTTTTTTTATATTTTGTTTTGTGTTATTTTTTTCTTTAACATCCCTAA
AATTTTCCTTACATGTTTTACTAGCCAGATTTTTCCTCCTCTCCTGACTACTCCCAGTCATAGCTGTCCC
TCTTCTCTTATGAAGATCCCTCGACCTGCAGCCCAAGCTTGGCGTAATCATGGTCATAGCTGTTTCCTGT
GTGAAATTGTTATCCGCTCACAATTCCACACAACATACGAGCCGGAAGCATAAAGTGTAAAGCCTGGGGT
GCCTAATGAGTGAGCTAACTCACATTAATTGCGTTGCGCTCACTGCCCGCTTTCCAGTCGGGAAACCTGT
CGTGCCAGCGGATCCGCATCTCAATTAGTCAGCAACCATAGTCCCGCCCCTAACTCCGCCCATCCCGCCC

CTAACTCCGCCCAGTTCCGCCCATTCTCCGCCCCATGGCTGACTAATTTTTTTTATTTATGCAGAGGCCG
AGGCCGCCTCGGCCTCTGAGCTATTCCAGAAGTAGTGAGGAGGCTTTTTTGGAGGCCTAGGCTTTTGCAA
AAAGCTAACTTGTTTATTGCAGCTTATAATGGTTACAAATAAAGCAATAGCATCACAAATTTCACAAATA
AAGCATTTTTTTCACTGCATTCTAGTTGTGGTTTGTCCAAACTCATCAATGTATCTTATCATGTCTGGAT
CCGCTGCATTAATGAATCGGCCAACGCGCGGGGAGAGGCGGTTTGCGTATTGGGCGCTCTTCCGCTTCCT
CGCTCACTGACTCGCTGCGCTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAAGGCCAGCAAAAGGCCAGG
AACCGTAAAAAGGCCGCGTTGCTGGCGTTTTTCCATAGGCTCCGCCCCCCTGACGAGCATCACAAAAATC
GACGCTCAAGTCAGAGGTGGCGAAACCCGACAGGACTATAAAGATACCAGGCGTTTCCCCCTGGAAGCTC
CCTCGTGCGCTCTCCTGTTCCGACCCTGCCGCTTACCGGATACCTGTCCGCCTTTCTCCCTTCGGGAAGC
GTGGCGCTTTCTCAATGCTCACGCTGTAGGTATCTCAGTTCGGTGTAGGTCGTTCGCTCCAAGCTGGGCT
GTGTGCACGAACCCCCCGTTCAGCCCGACCGCTGCGCCTTATCCGGTAACTATCGTCTTGAGTCCAACCC
GGTAAGACACGACTTATCGCCACTGGCAGCAGCCACTGGTAACAGGATTAGCAGAGCGAGGTATGTAGGC
GGTGCTACAGAGTTCTTGAAGTGGTGGCCTAACTACGGCTACACTAGAAGGACAGTATTTGGTATCTGCG
CTCTGCTGAAGCCAGTTACCTTCGGAAAAAGAGTTGGTAGCTCTTGATCCGGCAAACAAACCACCGCTGG
TAGCGGTGGTTTTTTTGTTTGCAAGCAGCAGATTACGCGCAGAAAAAAAGGATCTCAAGAAGATCCTTTG
ATCTTTTCTACGGGGTCTGACGCTCAGTGGAACGAAAACTCACGTTAAGGGATTTTGGTCATGAGATTAT
CAAAAAGGATCTTCACCTAGATCCTTTTAAATTAAAAATGAAGTTTTAAATCAATCTAAAGTATATATGA
GTAAACTTGGTCTGACAGTTACCAATGCTTAATCAGTGAGGCACCTATCTCAGCGATCTGTCTATTTCGT
TCATCCATAGTTGCCTGACTCCCCGTCGTGTAGATAACTACGATACGGGAGGGCTTACCATCTGGCCCCA
GTGCTGCAATGATACCGCGAGACCCACGCTCACCGGCTCCAGATTTATCAGCAATAAACCAGCCAGCCGG
AAGGGCCGAGCGCAGAAGTGGTCCTGCAACTTTATCCGCCTCCATCCAGTCTATTAATTGTTGCCGGGAA
GCTAGAGTAAGTAGTTCGCCAGTTAATAGTTTGCGCAACGTTGTTGCCATTGCTACAGGCATCGTGGTGT
CACGCTCGTCGTTTGGTATGGCTTCATTCAGCTCCGGTTCCCAACGATCAAGGCGAGTTACATGATCCCC
CATGTTGTGCAAAAAAGCGGTTAGCTCCTTCGGTCCTCCGATCGTTGTCAGAAGTAAGTTGGCCGCAGTG
TTATCACTCATGGTTATGGCAGCACTGCATAATTCTCTTACTGTCATGCCATCCGTAAGATGCTTTTCTG
TGACTGGTGAGTACTCAACCAAGTCATTCTGAGAATAGTGTATGCGGCGACCGAGTTGCTCTTGCCCGGC
GTCAATACGGGATAATACCGCGCCACATAGCAGAACTTTAAAAGTGCTCATCATTGGAAAACGTTCTTCG
GGGCGAAAACTCTCAAGGATCTTACCGCTGTTGAGATCCAGTTCGATGTAACCCACTCGTGCACCCAACT
GATCTTCAGCATCTTTTACTTTCACCAGCGTTTCTGGGTGAGCAAAAACAGGAAGGCAAAATGCCGCAAA
AAAGGGAATAAGGGCGACACGGAAATGTTGAATACTCATACTCTTCCTTTTTCAATATTATTGAAGCATT
TATCAGGGTTATTGTCTCATGAGCGGATACATATTTGAATGTATTTAGAAAAATAAACAAATAGGGGTTC
CGCGCACATTTCCCCGAAAAGTGCCACCTG

SEQ ID NO: 2 (pHWX):
CAGCTGGTTCTTTCCGCCTCAGAAGCCATAGAGCCCACCGCATCCCCAGCATGCCTGCTATTGTCTTCCC
AATCCTCCCCCTTGCTGTCCTGCCCCACCCCACCCCCCAGAATAGAATGACACCTACTCAGACAATGCGA
TGCAATTTCCTCATTTTATTAGGAAAGGACAGTGGGAGTGGCACCTTCCAGGGTCAAGGAAGGCACGGGG
GAGGGGCAAACAACAGATGGCTGGCAACTAGAAGGCACAGTCGAGGCTGATCAGCGAGCTCTAGCATTTA
GGTGACCGCCGGGAGGGCGTCCCCGGCCCGGCGCTGCTCCCGCGTGTGTCCTGGGGTTGACCAGAGGGCC

CCGGGCGCTCCGTGTGTGGCTGCGATGGTGGCGTTTTTGGGGACAGGTGTCCGTGTCGCGCGTCGCCTGG
GCCGGCGGCGTGGTCGGTGACGCGACCTCCCGGCCCCGGGGGAGGTATATCTTTCGCTCCGAGTCGGCAT
TTTGGGCCGCCGGGTTATTGGAGACGGGGTCGACCTGCAGACTGGCTGTGTATAAGGGAGCCTGACATTT
ATATTCCCCAGAACATCAGGTTAATGGCGTTTTTGATGTCATTTTCGCGGTGGCTGAGATCAGCCACTTC
TTCCCCGATAACGGAAACCGGCACACTGGCCATATCGGTGGTCATCATGCGCCAGCTTTCATCCCCGATA
TGCACCACCGGGTAAAGTTCACGGGAGACTTTATCTGACAGCAGACGTGCACTGGCCAGGGGGATCACCA
TCCGTCGCCCGGGCGTGTCAATAATATCACTCTGTACATCCACAAACAGACGATAACGGCTCTCTCTTTT
ATAGGTGTAAACCTTAAACTGCATTTCACCAGCCCCTGTTCTCGTCAGCAAAAGAGCCGTTCATTTCAAT
AAACCGGGCGACCTCAGCCATCCCTTCCTGATTTTCCGCTTTCCAGCGTTCGGCACGCAGACGACGGGCT
TCATTCTGCATGGTTGTGCTTACCAGACCGGAGATATTGACATCATATATGCCTTGAGCAACTGATAGCT
GTCGCTGTCAACTGTCACTGTAATACGCTGCTTCATAGCATACCTCTTTTTGACATACTTCGGGTATACA
TATCAGTATATATTCTTATACCGCAAAAATCAGCGCGCAAATACGCATACTGTTATCTGGCTTTTAGTAA
GCCGGATCCACGCGTCGTCTCCTCCCCCCCAACTTCGGAGGTCGACCAGTACTCCGGTTAACTGCTAGAC
CCTATAGTGAGTCGTATTAATTTCGATAAGCCAGTAAGCAGTGGGTTCTCTAGTTAGCCAGAGAGCTCTG
CTTATATAGACCTCCCACCGTACACGCCTACCGCCCATTTGCGTCAATGGGGCGGAGTTGTTACGACATT
TTGGAAAGTCCCGTTGATTTTGGTGCCAAAACAAACTCCCATTGACGTCAATGGGGTGGAGACTTGGAAA
TCCCCGTGAGTCAAACCGCTATCCACGCCCATTGATGTACTGCCAAAACCGCATCACCATGGTAATAGCG
ATGACTAATACGTAGATGTACTGCCAAGTAGGAAAGTCCCATAAGGTCATGTACTGGGCATAATGCCAGG
CGGGCCATTTACCGTCATTGACGTCAATAGGGGGCGTACTTGGCATATCGACGTCAGGTGGCACTTTTCG
GGGAAATGTGCGCGGAACCCCTATTTGTTTATTTTTCTAAATACATTCAAATATGTATCCGCTCATGAGA
CAATAACCCTGATAAATGCTTCAATAATATTGAAAAAGGAAGAGTATGAGTATTCAACATTTCCGTGTCG
CCCTTATTCCCTTTTTTGCGGCATTTTGCCTTCCTGTTTTTGCTCACCCAGAAACGCTGGTGAAAGTAAA
AGATGCTGAAGATCAGTTGGGTGCACGAGTGGGTTACATCGAACTGGATCTCAACAGCGGTAAGATCCTT
GAGAGTTTTCGCCCCGAAGAACGTTTTCCAATGATGAGCACTTTTAAAGTTCTGCTATGTGGCGCGGTAT
TATCCCGTATTGACGCCGGGCAAGAGCAACTCGGTCGCCGCATACACTATTCTCAGAATGACTTGGTTGA
GTACTCACCAGTCACAGAAAAGCATCTTACGGATGGCATGACAGTAAGAGAATTATGCAGTGCTGCCATA
ACCATGAGTGATAACACTGCGGCCAACTTACTTCTGACAACGATCGGAGGACCGAAGGAGCTAACCGCTT
TTTTGCACAACATGGGGGATCATGTAACTCGCCTTGATCGTTGGGAACCGGAGCTGAATGAAGCCATACC
AAACGACGAGCGTGACACCACGATGCCTGTAGCAATGGCAACAACGTTGCGCAAACTATTAACTGGCGAA
CTACTTACTCTAGCTTCCCGGCAACAATTAATAGACTGGATGGAGGCGGATAAAGTTGCAGGACCACTTC
TGCGCTCGGCCCTTCCGGCTGGCTGGTTTATTGCTGATAAATCTGGAGCCGGTGAGCGTGGTTCTCGCGG
TATCATTGCAGCACTGGGGCCAGATGGTAAGCCCTCCCGTATCGTAGTTATCTACACGACGGGGAGTCAG
GCAACTATGGATGAACGAAATAGACAGATCGCTGAGATAGGTGCCTCACTGATTAAGCATTGGTAACTGT
CAGACCAAGTTTACTCATATATACTTTAGATTGATTTAAAACTTCATTTTTAATTTAAAAGGATCTAGGT
GAAGATCCTTTTTGATAATCTCATGACCAAAATCCCTTAACGTGAGTTTTCGTTCCACTGAGCGTCAGAC
CCCGTAGAAAAGATCAAAGGATCTTCTTGAGATCCTTTTTTTCTGCGCGTAATCTGCTGCTTGCAAACAA
AAAAACCACCGCTACCAGCGGTGGTTTGTTTGCCGGATCAAGAGCTACCAACTCTTTTTCCGAAGGTAAC
TGGCTTCAGCAGAGCGCAGATACCAAATACTGTTCTTCTAGTGTAGCCGTAGTTAGGCCACCACTTCAAG
AACTCTGTAGCACCGCCTACATACCTCGCTCTGCTAATCCTGTTACCAGTGGCTGCTGCCAGTGGCGATA
AGTCGTGTCTTACCGGGTTGGACTCAAGACGATAGTTACCGGATAAGGCGCAGCGGTCGGGCTGAACGGG

GGGTTCGTGCACACAGCCCAGCTTGGAGCGAACGACCTACACCGAACTGAGATACCTACAGCGTGAGCTA
TGAGAAAGCGCCACGCTTCCCGAAGGGAGAAAGGCGGACAGGTATCCGGTAAGCGGCAGGGTCGGAACAG
GAGAGCGCACGAGGGAGCTTCCAGGGGGAAACGCCTGGTATCTTTATAGTCCTGTCGGGTTTCGCCACCT
CTGACTTGAGCGTCGATTTTTGTGATGCTCGTCAGGGGGGCGGAGCCTATGGAAAAACGCCAGCAACGCG
GCCTTTTTACGGTTCCTGGCCTTTTGCTGGCCTTTTGCTCACATGTTCTTTCCTGCGTTATCCCCTGATT
CTGTGGATAACCGTATTACCGCCTTTGAGTGAGCTGATACCGCTCGCCGCAGCCGAACGACCGAGCGCAG
CGAGTCAGTGAGCGAGGAAGCGGAAGagcgcccaatacgcaaaccgcctctccccgcgcgttggccgatt
cattaatg

SEQ ID NO: 3 (Influenza A virus (A/California/04/2009(H1N1)) seg-
ment 1 polymerase PB2 (PB2) gene; FJ969516.1 Influenza A virus
(A/California/04/2009(H1N1)) segment 1 polymerase PB2 (PB2) gene,
complete cds)
ATGGAGAGAATAAAAGAACTGAGAGATCTAATGTCGCAGTCCCGCACTCGCGAGATACTCACTAAGACCA
CTGTGGACCATATGGCCATAATCAAAAAGTACACATCAGGAAGGCAAGAGAAGAACCCCGCACTCAGAAT
GAAGTGGATGATGGCAATGAGATACCCAATTACAGCAGACAAGAGAATAATGGACATGATTCCAGAGAGG
AATGAACAAGGACAAACCCTCTGGAGCAAAACAAACGATGCTGGATCAGACCGAGTGATGGTATCACCTC
TGGCCGTAACATGGTGGAATAGGAATGGCCCAACAACAAGTACAGTTCATTACCCTAAGGTATATAAAAC
TTATTTCGAAAAGGTCGAAAGGTTGAAACATGGTACCTTCGGCCCTGTCCACTTCAGAAATCAAGTTAAA
ATAAGGAGGAGAGTTGATACAAACCCTGGCCATGCAGATCTCAGTGCCAAGGAGGCACAGGATGTGATTA
TGGAAGTTGTTTTCCCAAATGAAGTGGGGGCAAGAATACTGACATCAGAGTCACAGCTGGCAATAACAAA
AGAGAAGAAAGAAGAGCTCCAGGATTGTAAAATTGCTCCCTTGATGGTGGCGTACATGCTAGAAAGAGAA
TTGGTCCGTAAAACAAGGTTTCTCCCAGTAGCCGGCGGAACAGGCAGTGTTTATATTGAAGTGTTGCACT
TAACCCAAGGGACGTGCTGGGAGCAGATGTACACTCCAGGAGGAGAAGTGAGAAATGATGATGTTGACCA
AAGTTTGATTATCGCTGCTAGAAACATAGTAAGAAGAGCAGCAGTGTCAGCAGACCCATTAGCATCTCTC
TTGGAAATGTGCCACAGCACACAGATTGGAGGAGTAAGGATGGTGGACATCCTTAGACAGAATCCAACTG
AGGAACAAGCCGTAGACATATGCAAGGCAGCAATAGGGTTGAGGATTAGCTCATCTTTCAGTTTTGGTGG
GTTCACTTTCAAAAGGACAAGCGGATCATCAGTCAAGAAAGAAGAAGAAGTGCTAACGGGCAACCTCCAA
ACACTGAAAATAAGAGTACATGAAGGGTATGAAGAATTCACAATGGTTGGGAGAAGAGCAACAGCTATTC
TCAGAAAGGCAACCAGGAGATTGATCCAGTTGATAGTAAGCGGGAGAGACGAGCAGTCAATTGCTGAGGC
AATAATTGTGGCCATGGTATTCTCACAGGAGGATTGCATGATCAAGGCAGTTAGGGGCGATCTGAACTTT
GTCAATAGGGCAAACCAGCGACTGAACCCCATGCACCAACTCTTGAGGCATTTCCAAAAAGATGCAAAAG
TGCTTTTCCAGAACTGGGGAATTGAATCCATCGACAATGTGATGGGAATGATCGGAATACTGCCCGACAT
GACCCCAAGCACGGAGATGTCGCTGAGAGGGATAAGAGTCAGCAAAATGGGAGTAGATGAATACTCCAGC
ACGGAGAGAGTGGTAGTGAGTATTGACCGATTTTTAAGGGTTAGAGATCAAAGAGGGAACGTACTATTGT
CTCCCGAAGAAGTCAGTGAAACGCAAGGAACTGAGAAGTTGACAATAACTTATTCGTCATCAATGATGTG
GGAGATCAATGGCCCTGAGTCAGTGCTAGTCAACACTTATCAATGGATAATCAGGAACTGGGAAATTGTG
AAAATTCAATGGTCACAAGATCCCACAATGTTATACAACAAAATGGAATTTGAACCATTTCAGTCTCTTG
TCCCTAAGGCAACCAGAAGCCGGTACAGTGGATTCGTAAGGACACTGTTCCAGCAAATGCGGGATGTGCT
TGGGACATTTGACACTGTCCAAATAATAAAACTTCTCCCCTTTGCTGCTGCCCCACCAGAACAGAGTAGG

ATGCAATTTTCCTCATTGACTGTGAATGTGAGAGGATCAGGGTTGAGGATACTGGTAAGAGGCAATTCTC
CAGTATTCAATTACAACAAGGCAACCAAACGACTTACAGTTCTTGGAAAGGATGCAGGTGCATTGACTGA
AGATCCAGATGAAGGCACATCTGGGGTGGAGTCTGCTGTCCTGAGAGGATTTCTCATTTTGGGCAAAGAA
GACAAGAGATATGGCCCAGCATTAAGCATCAATGAACTGAGCAATCTTGCAAAAGGAGAGAAGGCTAATG
TGCTAATTGGGCAAGGGGACGTAGTGTTGGTAATGAAACGAAAACGGGACTCTAGCATACTTACTGACAG
CCAGACAGCGACCAAAAGAATTCGGATGGCCATCAATTAG

SEQ ID NO: 4 (Influenza A virus (A/California/04/2009(H1N1)) seg-
ment 2 polymerase PB1 (PB1) gene; MK159411.1 Influenza A virus
(A/California/04/2009(H1N1)) segment 2 polymerase PB1 (PB1) gene,
complete cds; and nonfunctional PB1-F2 protein (PB1-F2) gene, com-
plete sequence)
AGCAAAAGCAGGCAAACCATTTGAATGGATGTCAATCCGACTCTACTTTTCCTAAAAATTCCAGCGCAAA
ATGCCATAAGCACCACATTCCCTTATACTGGAGATCCTCCATACAGCCATGGAACAGGAACAGGATACAC
CATGGACACAGTAAACAGAACACACCAATACTCAGAAAAGGGAAAGTGGACGACAAACACAGAGACTGGT
GCACCCCAGCTCAACCCGATTGATGGACCACTACCTGAGGATAATGAACCAAGTGGGTATGCACAAACAG
ACTGTGTTCTAGAGGCTATGGCTTTCCTTGAAGAATCCCACCCAGGAATATTTGAGAATTCATGCCTTGA
AACAATGGAAGTTGTTCAACAAACAAGGGTAGATAAACTAACTCAAGGTCGCCAGACTTATGATTGGACA
TTAAACAGAAATCAACCGGCAGCAACTGCATTGGCCAACACCATAGAAGTCTTTAGATCGAATGGCCTAA
CAGCTAATGAGTCAGGAAGGCTAATAGATTTCTTAAAGGATGTAATGGAATCAATGAACAAAGAGGAAAT
AGAGATAACAACCCACTTTCAAAGAAAAAGGAGAGTAAGAGACAACATGACCAAGAAGATGGTCACGCAA
AGAACAATAGGGAAGAAAAAACAAAGACTGAATAAGAGAGGCTATCTAATAAGAGCACTGACATTAAATA
CGATGACCAAAGATGCAGAGAGAGGCAAGTTAAAAAGAAGGGCTATCGCAACACCTGGGATGCAGATTAG
AGGTTTCGTATACTTTGTTGAAACTTTAGCTAGGAGCATTTGCGAAAAGCTTGAACAGTCTGGGCTCCCA
GTAGGGGGCAATGAAAAGAAGGCCAAACTGGCAAATGTTGTGAGAAAGATGATGACTAATTCACAAGACA
CAGAGATTTCTTTCACAATCACTGGGGACAACACTAAGTGGAATGAAAATCAAAATCCTCGAATGTTCCT
GGCGATGATTACATATATCACCAGAAATCAACCCGAGTGGTTCAGAAACATCCTGAGCATGGCACCCATA
ATGTTCTCAAACAAAATGGCAAGACTAGGGAAAGGGTACATGTTCGAGAGTAAAAGAATGAAGATTCGAA
CACAAATACCAGCAGAAATGCTAGCAAGCATTGACCTGAAGTACTTCAATGAATCAACAAAGAAGAAAAT
TGAGAAAATAAGGCCTCTTCTAATAGATGGCACAGCATCACTGAGTCCTGGGATGATGATGGGCATGTTC
AACATGCTAAGTACGGTCTTGGGAGTCTCGATACTGAATCTTGGACAAAAGAAATACACCAAGACAATAT
ACTGGTGGGATGGGCTCCAATCATCCGACGATTTTGCTCTCATAGTGAATGCACCAAACCATGAGGGAAT
ACAAGCAGGAGTGGACAGATTCTACAGGACCTGCAAGTTAGTGGGAATCAACATGAGCAAAAAGAAGTCC
TATATAAATAAGACAGGGACATTTGAATTCACAAGCTTTTTTTATCGCTATGGATTTGTGGCTAATTTTA
GCATGGAGCTACCCAGCTTTGGAGTGTCTGGAGTAAATGAATCAGCTGACATGAGTATTGGAGTAACAGT
GATAAAGAACAACATGATAAACAATGACCTTGGACCTGCAACGGCCCAGATGGCTCTTCAATTGTTCATC
AAAGACTACAGATACACATATAGGTGCCATAGGGGAGACACACAAATTCAGACGAGAAGATCATTTGAGT
TAAAGAAGCTGTGGGATCAAACCCAATCAAAAGTAGGGCTATTAGTATCAGATGGAGGACCAAACTTATA
CAATATACGGAATCTTCACATTCCTGAAGTCTGCTTAAAATGGGAGCTAATGGATGATGATTATCGGGGA

AGACTTTGTAATCCCCTGAATCCCTTTGTCAGTCATAAAGAGATTGATTCTGTAAACAATGCTGTGGTAA
TGCCAGCCCATGGTCCAGCCAAAAGCATGGAATATGATGCCGTTGCAACTACACATTCCTGGATTCCCAA
GAGGAATCGTTCTATTCTCAACACAAGCCAAAGGGGAATTCTTGAGGATGAACAGATGTACCAGAAGTGC
TGCAATCTATTCGAGAAATTTTTCCCTAGCAGTTCATATAGGAGACCGGTTGGAATTTCTAGCATGGTGG
AGGCCATGGTGTCTAGGGCCCGGATTGATGCCAGGGTCGACTTCGAGTCTGGACGGATCAAGAAAGAAGA
GTTCTCTGAGATCATGAAGATCTGTTCCACCATTGAAGAACTCAGACGGCAAAAATAATGAATTTAACTT
GTCCTTCATGAAAAAATGCCTTGTTTCTACT

SEQ ID NO: 5 (Influenza A virus (A/California/04/2009(H1N1)) seg-
ment 3 polymerase PA (PA) gene; FJ966081.1 Influenza A virus
(A/California/04/2009(H1N1)) segment 3 polymerase PA (PA) gene,
complete cds)
ATGGAAGACTTTGTGCGACAATGCTTCAATCCAATGATCGTCGAGCTTGCGGAAAAGGCAATGAAAGAAT
ATGGGGAAGATCCGAAAATCGAAACTAACAAGTTTGCTGCAATATGCACACATTTGGAAGTTTGTTTCAT
GTATTCGGATTTCCATTTCATCGACGAACGGGGTGAATCAATAATTGTAGAATCTGGTGACCCGAATGCA
CTATTGAAGCACCGATTTGAGATAATTGAAGGAAGAGACCGAATCATGGCCTGGACAGTGGTGAACAGTA
TATGTAACACAACAGGGGTAGAGAAGCCTAAATTTCTTCCTGATTTGTATGATTACAAAGAGAACCGGTT
CATTGAAATTGGAGTAACACGGAGGGAAGTCCACATATATTACCTAGAGAAAGCCAACAAAATAAAATCT
GAGAAGACACACATTCACATCTTTTCATTCACTGGAGAGGAGATGGCCACCAAAGCGGACTACACCCTTG
ACGAAGAGAGCAGGGCAAGAATCAAAACTAGGCTTTTCACTATAAGACAAGAAATGGCCAGTAGGAGTCT
ATGGGATTCCTTTCGTCAGTCCGAAAGAGGCGAAGAGACAATTGAAGAAAAATTTGAGATTACAGGAACT
ATGCGCAAGCTTGCCGACCAAAGTCTCCCACCGAACTTCCCCAGCCTTGAAAACTTTAGAGCCTATGTAG
ATGGATTCGAGCCGAACGGCTGCATTGAGGGCAAGCTTTCCCAAATGTCAAAAGAAGTGAACGCCAAAAT
TGAACCATTCTTGAGGACGACACCACGCCCCCTCAGATTGCCTGATGGGCCTCTTTGCCATCAGCGGTCA
AAGTTCCTGCTGATGGATGCTCTGAAATTAAGTATTGAAGACCCGAGTCACGAGGGGGAGGGAATACCAC
TATATGATGCAATCAAATGCATGAAGACATTCTTTGGCTGGAAAGAGCCTAACATAGTCAAACCACATGA
GAAAGGCATAAATCCCAATTACCTCATGGCTTGGAAGCAGGTGCTAGCAGAGCTACAGGACATTGAAAAT
GAAGAGAAGATCCCAAGGACAAAGAACATGAAGAGAACAAGCCAATTGAAGTGGGCACTCGGTGAAAATA
TGGCACCAGAAAAAGTAGACTTTGATGACTGCAAAGATGTTGGAGACCTTAAACAGTATGACAGTGATGA
GCCAGAGCCCAGATCTCTAGCAAGCTGGGTCCAAAATGAATTCAATAAGGCATGTGAATTGACTGATTCA
AGCTGGATAGAACTTGATGAAATAGGAGAAGATGTTGCCCCGATTGAACATATCGCAAGCATGAGGAGGA
ACTATTTTACAGCAGAAGTGTCCCACTGCAGGGCTACTGAATACATAATGAAGGGAGTGTACATAAATAC
GGCCTTGCTCAATGCATCCTGTGCAGCCATGGATGACTTTCAGCTGATCCCAATGATAAGCAAATGTAGG
ACCAAAGAAGGAAGACGGAAAACAAACCTGTATGGGTTCATTATAAAAGGAAGGTCTCATTTGAGAAATG
ATACTGATGTGGTGAACTTTGTAAGTATGGAGTTCTCACTCACTGACCCGAGACTGGAGCCACACAAATG
GGAAAAATACTGTGTTCTTGAAATAGGAGACATGCTCTTGAGGACTGCGATAGGCCAAGTGTCGAGGCCC
ATGTTCCTATATGTGAGAACCAATGGAACCTCCAAGATCAAGATGAAATGGGGCATGGAAATGAGGCGCT
GCCTTCTTCAGTCTCTTCAGCAGATTGAGAGCATGATTGAGGCCGAGTCTTCTGTCAAAGAGAAAGACAT
GACCAAGGAATTCTTTGAAAACAAATCGGAAACATGGCCAATCGGAGAGTCACCCAGGGGAGTGGAGGAA
GGCTCTATTGGAAAGTGTGCAGGACCTTACTGGCAAAATCTGTATTCAACAGTCTATATGCGTCTCCAC

AACTTGAGGGGTTTTCGGCTGAATCTAGAAAATTGCTTCTCATTGTTCAGGCACTTAGGGACAACCTGGA
ACCTGGAACCTTCGATCTTGGGGGGCTATATGAAGCAATCGAGGAGTGCCTGATTAATGATCCCTGGGTT
TTGCTTAATGCATCTTGGTTCAACTCCTTCCTCACACATGCACTGAAGTAG

SEQ ID NO: 6 (Influenza A virus (A/California/04/2009(H1N1)) seg-
ment 5 nucleocapsid protein (NP) gene; FJ966083.1 Influenza A virus
(A/California/04/2009(H1N1)) segment 5 nucleocapsid protein (NP)
gene, complete cds)
ATGGCGTCTCAAGGCACCAAACGATCATATGAACAAATGGAGACTGGTGGGGAGCGCCAGGATGCCACAG
AAATCAGAGCATCTGTCGGAAGAATGATTGGTGGAATCGGGAGATTCTACATCCAAATGTGCACTGAACT
CAAACTCAGTGATTATGATGGACGACTAATCCAGAATAGCATAACAATAGAGAGGATGGTGCTTTCTGCT
TTTGATGAGAGAAGAAATAAATACCTAGAAGAGCATCCCAGTGCTGGGAAGGACCCTAAGAAAACAGGAG
GACCCATATATAGAAGAGTAGACGGAAAGTGGATGAGAGAACTCATCCTTTATGACAAAGAAGAAATAAG
GAGAGTTTGGCGCCAAGCAAACAATGGCGAAGATGCAACAGCAGGTCTTACTCATATCATGATTTGGCAT
TCCAACCTGAATGATGCCACATATCAGAGAACAAGAGCGCTTGTTCGCACCGGAATGGATCCCAGAATGT
GCTCTCTAATGCAAGGTTCAACACTTCCCAGAAGGTCTGGTGCCGCAGGTGCTGCGGTGAAAGGAGTTGG
AACAATAGCAATGGAGTTAATCAGAATGATCAAACGTGGAATCAATGACCGAAATTTCTGGAGGGGTGAA
AATGGACGAAGGACAAGGGTTGCTTATGAAAGAATGTGCAATATCCTCAAAGGAAAATTTCAAACAGCTG
CCCAGAGGGCAATGATGGATCAAGTAAGAGAAAGTCGAAACCCAGGAAACGCTGAGATTGAAGACCTCAT
TTTCCTGGCACGGTCAGCACTCATTCTGAGGGGATCAGTTGCACATAAATCCTGCCTGCCTGCTTGTGTG
TATGGGCTTGCAGTAGCAAGTGGGCATGACTTTGAAAGGGAAGGGTACTCACTGGTCGGGATAGACCCAT
TCAAATTACTCCAAAACAGCCAAGTGGTCAGCCTGATGAGACCAAATGAAAACCCAGCTCACAAGAGTCA
ATTGGTGTGGATGGCATGCCACTCTGCTGCATTTGAAGATTTAAGAGTATCAAGTTTCATAAGAGGAAAG
AAAGTGATTCCAAGAGGAAAGCTTTCCACAAGAGGGGTCCAGATTGCTTCAAATGAGAATGTGGAAACCA
TGGACTCCAATACCCTGGAACTGAGAAGCAGATACTGGGCCATAAGGACCAGGAGTGGAGGAAATACCAA
TCAACAAAAGGCATCCGCAGGCCAGATCAGTGTGCAGCCTACATTCTCAGTGCAGCGGAATCTCCCTTTT
GAAAGAGCAACCGTTATGGCAGCATTCAGCGGGAACAATGAAGGACGGACATCCGACATGCGAACAGAAG
TTATAAGAATGATGGAAAGTGCAAAGCCAGAAGATTTGTCCTTCCAGGGGCGGGGAGTCTTCGAGCTCTC
GGACGAAAAGGCAACGAACCCGATCGTGCCTTCCTTTGACATGAGTAATGAAGGGTCTTATTTCTTCGGA
GACAATGCAGAGGAGTATGACAGTTGA

SEQ ID NO: 7 (Influenza A virus (A/California/04/2009(H1N1)) seg-
ment 7 matrix protein 2 (M2) and matrix protein 1 (M1) genes;
FJ969513.1 Influenza A virus (A/California/04/2009(H1N1)) segment
7 matrix protein 2 (M2) and matrix protein 1 (M1) genes, complete
cds)
ATGAGTCTTCTAACCGAGGTCGAAACGTACGTTCTTTCTATCATCCCGTCAGGCCCCCTCAAAGCCGAGA
TCGCGCAGAGACTGGAAAGTGTCTTTGCAGGAAAGAACACAGATCTTGAGGCTCTCATGGAATGGCTAAA
GACAAGACCAATCTTGTCACCTCTGACTAAGGGAATTTTAGGATTTGTGTTCACGCTCACCGTGCCCAGT
GAGCGAGGACTGCAGCGTAGACGCTTTGTCCAAAATGCCCTAAATGGGAATGGGGACCCGAACAACATGG

ATAGAGCAGTTAAACTATACAAGAAGCTCAAAAGAGAAATAACGTTCCATGGGGCCAAGGAGGTGTCACT
AAGCTATTCAACTGGTGCACTTGCCAGTTGCATGGGCCTCATATACAACAGGATGGGAACAGTGACCACA
GAAGCTGCTTTTGGTCTAGTGTGTGCCACTTGTGAACAGATTGCTGATTCACAGCATCGGTCTCACAGAC
AGATGGCTACTACCACCAATCCACTAATCAGGCATGAAAACAGAATGGTGCTGGCTAGCACTACGGCAAA
GGCTATGGAACAGATGGCTGGATCGAGTGAACAGGCAGCGGAGGCCATGGAGGTTGCTAATCAGACTAGG
CAGATGGTACATGCAATGAGAACTATTGGGACTCATCCTAGCTCCAGTGCTGGTCTGAAAGATGACCTTC
TTGAAAATTTGCAGGCCTACCAGAAGCGAATGGGAGTGCAGATGCAGCGATTCAAGTGATCCTCTCGTCA
TTGCAGCAAATATCATTGGGATCTTGCACCTGATATTGTGGATTACTGATCGTCTTTTTTTCAAATGTAT
TTATCGTCGCTTTAAATACGGTTTGAAAAGAGGGCCTTCTACGGAAGGAGTGCCTGAGTCCATGAGGGAA
GAATATCAACAGGAACAGCAGAGTGCTGTGGATGTTGACGATGGTCATTTTGTCAACATAGAGCTAGAGT
AA

SEQ ID NO: 8 (Influenza A virus (A/New Caledonia/20/1999(H1N1))
segment 4 HA gene; CY031336.1 Influenza A virus (A/New Caledo-
nia/20/1999(H1N1)) segment 4 sequence)
AGCAAAAGCAGGGGAAAATAAAAACAACCAAAATGAAAGCAAAACTACTGGTCCTGTTATGTACATTTAC
AGCTACATATGCAGACACAATATGTATAGGCTACCATGCCAACAACTCAACCGACACTGTTGACACAGTA
CTTGAGAAGAATGTGACAGTGACACACTCTGTCAACCTACTTGAGGACAGTCACAATGGAAAACTATGTC
TACTAAAAGGAATAGCCCCACTACAATTGGGTAATTGCAGCGTTGCCGGATGGATCTTAGGAAACCCAGA
ATGCGAATTACTGATTTCCAAGGAATCATGGTCCTACATTGTAGAAACACCAAATCCTGAGAATGGAACA
TGTTACCCAGGGTATTTCGCCGACTATGAGGAACTGAGGGAGCAATTGAGTTCAGTATCTTCATTTGAGA
GATTCGAAATATTCCCCAAAGAAAGCTCATGGCCCACCCACACCGTAACCGGAGTATCAGCATCATGCTC
CCATAATGGGAAAAGCAGTTTTTACAGAAATTTGCTATGGCTGACGGGGAAGAATGGTTTGTACCCAAAC
CTGAGCAAGTCCTATGTAAACAACAAAGAGAAAGAAGTCCTTGTACTATGGGGTGTTCATCACCCGCCTA
ACATAGGGGACCAAAGGGCCCTCTATCATACAGAAAATGCTTATGTCTCTGTAGTGTCTTCACATTATAG
CAGAAGATTCACCCCAGAAATAGCCAAAAGACCCAAAGTAAGAGATCAGGAAGGAAGAATCAACTACTAC
TGGACTCTGCTGGAACCTGGGGATACAATAATATTTGAGGCAAATGGAAATCTAATAGCGCCATGGTATG
CTTTTGCACTGAGTAGAGGCTTTGGATCAGGAATCATCACCTCAAATGCACCAATGGATGAATGTGATGC
GAAGTGTCAAACACCTCAGGGAGCTATAAACAGCAGTCTTCCTTTCCAGAATGTACACCCAGTCACAATA
GGAGAGTGTCCAAAGTATGTCAGGAGTGCAAAATTAAGGATGGTTACAGGACTAAGGAACATCCCATCCA
TTCAATCCAGAGGTTTGTTTGGAGCCATTGCCGGTTTCATTGAAGGGGGGTGGACTGGAATGGTAGATGG
GTGGTATGGTTATCATCATCAGAATGAGCAAGGATCTGGCTATGCTGCAGATCAAAAAAGTACACAAAAT
GCCATTAACGGGATTACAAACAAGGTGAATTCTGTAATTGAGAAAATGAACACTCAATTCACAGCTGTGG
GCAAAGAATTCAACAAATTGGAAAGAAGGATGGAAAACTTAAATAAAAAAGTTGATGATGGGTTTCTAGA
CATTTGGACATATAATGCAGAATTGTTGGTTCTACTGGAAAATGAAAGGACTTTGGATTTCCATGACTCC
AATGTGAAGAATCTGTATGAGAAAGTAAAAAGCCAATTAAAGAATAATGCCAAAGAAATAGGAAACGGGT
GTTTTGAATTCTATCACAAGTGTAACAATGAATGCATGGAGAGTGTGAAAAATGGAACTTATGACTATCC
AAAATATTCCGAAGAATCAAAGTTAAACAGGGAGAAAATTGATGGAGTGAAATTGGAATCAATGGGAGTC
TATCAGATTCTGGCGATCTACTCAACTGTCGCCAGTTCCCTGGTTCTTTTGGTCTCCCTGGGGGCAATCA
GCTTCTGGATGTGTTCCAATGGGTCTTTGCAGTGTAGAATATGCATCTGAGACCAGAATTTCAGAAATAT

AAGAAAAAACACCCTTGTTTCTACT

SEQ ID NO: 9 (Influenza A virus (A/New Caledonia/20/1999(H1N1))
segment 6 NA gene; CY033624.1 Influenza A virus (A/New Caledo-
nia/20/1999(H1N1)) segment 6, complete sequence)
AATGAATCCAAATCAAAAAATAATAACCATTGGATCAATCAGTATAGCAATCGGAATAATTAGTCTAATG
TTGCAAATAGGAAATATTATTTCAATATGGGCTAGTCACTCAATCCAAACTGGAAGTCAAAACCACACTG
GAGTATGCAACCAAAGAATCATCACATATGAAAACAGCACCTGGGTGAATCACACATATGTTAATATTAA
CAACACTAATGTTGTTGCTGGAAAGGACAAAACTTCAGTGACATTGGCCGGCAATTCATCTCTTTGTTCT
ATCAGTGGATGGGCTATATACACAAAAGACAACAGCATAAGAATTGGCTCCAAAGGAGATGTTTTTGTCA
TAAGAGAACCTTTCATATCATGTTCTCACTTGGAATGCAGAACCTTTTTTCTGACCCAAGGTGCTCTATT
AAATGACAAACATTCAAATGGGACCGTTAAGGACAGAAGTCCTTATAGGGCCTTAATGAGCTGTCCTCTA
GGTGAAGCTCCGTCCCCATACAATTCAAAGTTTGAATCAGTTGCATGGTCAGCAAGCGCATGCCATGATG
GCATGGGCTGGTTAACAATCGGAATTTCTGGTCCAGACAATGGAGCTGTGGCTGTACTAAAATACAACGG
CATAATAACTGAAACCATAAAAAGTTGGAAAAAGCGAATATTAAGAACACAAGAGTCTGAATGTGTCTGT
GTGAACGGGTCATGTTTCACCATAATGACCGATGGCCCGAGTAATGGGGCCGCCTCGTACAAAATCTTCA
AGATCGAAAAGGGGAAGGTTACTAAATCAATAGAGTTGAATGCACCCAATTTTCATTATGAGGAATGTTC
CTGTTACCCAGACACTGGCACAGTGATGTGTGTATGCAGGGACAACTGGCATGGTTCAAATCGACCTTGG
GTGTCTTTTAATCAAAACCTGGATTATCAAATAGGATACATCTGCAGTGGGGTGTTCGGTGACAATCCGC
GTCCCAAAGATGGAGAGGGCAGCTGTAATCCAGTGACTGTTGATGGAGCAGACGGAGTAAAGGGGTTTTC
ATACAAATATGGTAATGGTGTTTGGATAGGAAGGACTAAAAGTAACAGACTTAGAAAGGGGTTTGAGATG
ATTTGGGATCCTAATGGATGGACAGATACCGACAGTGATTTCTCAGTGAAACAGGATGTTGTGGCAATAA
CTGATTGGTCAGGGTACAGCGGAAGTTTCGTTCAACATCCTGAGTTAACAGGATTGGACTGTATAAGACC
TTGCTTCTGGGTTGAGTTAGTCAGAGGACTGCCTAGAGAAAATACAACAATCTGGACTAGTGGGAGCAGC
ATTTCTTTTTGTGGCGTAAATAGTGATACTGCAAACTGGTCTTGGCCAGACGGTGCTGAGTTGCCGTTCA
CCATTGACAAGTAG

SEQ ID Nos: 3 to 9 are all available in the UNIPROT database.

## Claims

1. Influenza virus-like particles (VLPs), wherein the VLPs comprise:
- hemagglutinin (HA) protein and neuraminidase (NA) protein on the surface of the VLPs,
- a nucleoprotein (NP) ribonucleoprotein complex,
wherein the VLPs do not contain a ribonucleoprotein complex of at least one of PB1, PB2, and NS2.

2. VLPs according to claim 1, further comprising a matrix protein (M) ribonucleoprotein complex.

3. VLPs according to claim 1 or 2, wherein the HA protein is selected from the group of seasonal, non-pandemic HA proteins.

4. VLPs according to any one of claims 1 to 3, wherein the NA protein is selected from the group of seasonal, non-pandemic HA proteins.

5. VLPs according to any one of claims 1 to 4, further comprising a genetically modified M ribonucleoprotein complex, preferably wherein the M ribonucleoprotein complex contains viral RNA encoding an antigen of a non-influenza virus pathogen, an oncogene or a malignant tissue protein, especially wherein the M ribonucleoprotein complex contains viral RNA encoding a fusion protein containing an M protein and a protein or antigenic protein fragment of a non-influenza virus pathogen.

6. VLPs according to claim 5, wherein especially wherein the non-influenza virus pathogen is selected from a non-influenza virus, a gram-positive or gram-negative bacterium, a fungus, a protozoan, or a prion, preferably a non-influenza RNA virus, a DNA virus, or a retrovirus, especially wherein the virus is SARS-COV-1, SARS-COV-2, or Dengue virus.

7. VLPs according to any one of claims 1 to 6, wherein the VLPs comprise:
- HA protein and NA protein on the surface of the VLPs,
- a NP ribonucleoprotein complex, and, preferably an M ribonucleoprotein complex,
wherein the VLPs do not contain a ribonucleoprotein complex of at least two of PB1, PB2, NS1 and NS2; preferably wherein the VLPs do not contain a ribonucleoprotein complex of at least three of PB1, PB2, NS1 and NS2; even more preferred wherein the VLPs do not contain a ribonucleoprotein complex of NS1; especially wherein the VLPs do not contain a ribonucleoprotein complex of PB1, PB2, NS1 and NS2.

8. VLPs according to any one of claims 1 to 7, triggering inflammatory cell death (necroptosis) upon entry into a cell.

9. Vaccine comprising the VLPs according to any one of claims 1 to 8, and a pharmaceutically acceptable excipient.

10. Vaccine according to claim 9, wherein the vaccine is an intranasal vaccine, preferably an aerosol or nasal spray.

11. Pharmaceutical composition comprising the VLPs according to any one of claims 1 to 8.

12. Method for producing VLPs according to any one of claims 1 to 8, comprising the steps of
- providing unidirectional vectors for HA, NA, PA, PB1 and PB2;
- providing a bidirectional vector for NP,
- providing either a unidirectional or bidirectional vector for M,
- expressing the vectors in a recombinant cell system so as to obtain VLPs according to any one of claims 1 to 8.

13. Method according to claim 12, further comprising the steps of filling the VLPs into a final container and finishing the VLPs to a pharmaceutical composition ready for use for administration to human individuals.

14. Method according to claim 12 or 13, wherein the vectors are expressed in Madin-Darby Canine Kidney (MDCK) cells, African green monkey kidney cells (Vero) cells, 293 cells, 293T cells, porcine kidney (PK) cells, owl monkey kidney (OMK) cells, Madin-Darby bovine kidney (MDBK) cells, chicken embryo kidney (CEK) cells, chicken embryo fibroblasts, primary chick kidney cells, or cells isolated from the chorioallantoic membrane of embryonated chicken eggs, preferably in MDCK cells or Vero cells, especially in MDCK cells.

15. VLPs according to any one of claims 1 to 8, for medical use, preferably for preventing pathogen-caused diseases, especially for preventing influenza.


SEQUENCE LISTING

<110> ÖSTERREICHISCHE AGENTUR FÜR GESUNDHEIT UND
      ERNÄHRUNGSSICHERHEIT GMBH

<120> Influenza virus-like particles (VLPs)

<130> R 76406

<160> 9

<170> PatentIn version 3.5

<210> 1
<211> 4790
<212> DNA
<213> Artificial Sequence

<220>
<223> pCAGGS

<400> 1
gtcgacattg attattgact agttattaat agtaatcaat tacggggtca ttagttcata 60

gcccatatat ggagttccgc gttacataac ttacggtaaa tggcccgcct ggctgaccgc 120

ccaacgaccc ccgcccattg acgtcaataa tgacgtatgt tcccatagta acgccaatag 180

ggactttcca ttgacgtcaa tgggtggact atttacggta aactgcccac ttggcagtac 240

atcaagtgta tcatatgcca agtacgcccc ctattgacgt caatgacggt aaatggcccg 300

cctggcatta tgcccagtac atgaccttat gggactttcc tacttggcag tacatctacg 360

tattagtcat cgctattacc atgggtcgag gtgagcccca cgttctgctt cactctcccc 420

atctcccccc cctccccacc cccaattttg tatttattta ttttttaatt attttgtgca 480

gcgatggggg cggggggggg gggggcgcgc gccaggcggg gcggggcggg gcgaggggcg 540

gggcggggcg aggcggagag gtgcggcggc agccaatcag agcggcgcgc tccgaaagtt 600

tccttttatg gcgaggcggc ggcggcggcg gccctataaa aagcgaagcg cgcggcgggc 660

gggagtcgct gcgttgcctt cgccccgtgc cccgctccgc gccgcctcgc gccgcccgcc 720

ccggctctga ctgaccgcgt tactcccaca ggtgagcggg cgggacggcc cttctcctcc 780

gggctgtaat tagcgcttgg tttaatgacg gctcgtttct tttctgtggc tgcgtgaaag 840

ccttaaaggg ctccgggagg gccctttgtg cgggggggag cggctcgggg ggtgcgtgcg 900

tgtgtgtgtg cgtggggagc gccgcgtgcg gcccgcgctg cccggcggct gtgagcgctg 960

cgggcgcggc gcggggcttt gtgcgctccg cgtgtgcgcg aggggagcgc ggccgggggc 1020

ggtgccccgc ggtgcggggg ggctgcgagg ggaacaaagg ctgcgtgcgg ggtgtgtgcg 1080

tgggggggtg agcagggggt gtgggcgcgg cggtcgggct gtaacccccc cctgcacccc 1140

cctccccgag ttgctgagca cggcccggct tcgggtgcgg ggctccgtgc ggggcgtggc 1200

gcggggctcg ccgtgccggg cggggggtgg cggcaggtgg gggtgccggg cggggcgggg 1260

ccgcctcggg ccggggaggg ctcgggggag gggcgcggcg gccccggagc gccggcggct 1320

gtcgaggcgc ggcgagccgc agccattgcc ttttatggta atcgtgcgag agggcgcagg 1380

gacttccttt gtcccaaatc tggcggagcc gaaatctggg aggcgccgcc gcaccccctc 1440

tagcgggcgc gggcgaagcg gtgcggcgcc ggcaggaagg aaatgggcgg ggagggcctt 1500

cgtgcgtcgc cgcgccgccg tccccttctc catctccagc ctcggggctg ccgcaggggg 1560

acggctgcct tcggggggga cggggcaggg cggggttcgg cttctggcgt gtgaccggcg 1620

gctctagagc ctctgctaac catgttcatg ccttcttctt tttcctacag ctcctgggca 1680

acgtgctggt tgttgtgctg tctcatcatt ttggcaaaga attcctcgag gaattcactc 1740

ctcaggtgca ggctgcctat cagaaggtgg tggctggtgt ggccaatgcc ctggctcaca 1800

aataccactg agatcttttt ccctctgcca aaaattatgg ggacatcatg aagccccttg 1860

agcatctgac ttctggctaa taaaggaaat ttattttcat tgcaatagtg tgttggaatt 1920

ttttgtgtct ctcactcgga aggacatatg ggagggcaaa tcatttaaaa catcagaatg 1980

agtatttggt ttagagtttg gcaacatatg ccatatgctg gctgccatga acaaaggtgg 2040

ctataaagag gtcatcagta tatgaaacag ccccctgctg tccattcctt attccataga 2100

aaagccttga cttgaggtta gatttttttt atattttgtt ttgtgttatt tttttcttta 2160

acatccctaa aattttcctt acatgtttta ctagccagat ttttcctcct ctcctgacta 2220

ctcccagtca tagctgtccc tcttctctta tgaagatccc tcgacctgca gcccaagctt 2280

ggcgtaatca tggtcatagc tgtttcctgt gtgaaattgt tatccgctca caattccaca 2340

caacatacga gccggaagca taaagtgtaa agcctggggt gcctaatgag tgagctaact 2400

cacattaatt gcgttgcgct cactgcccgc tttccagtcg ggaaacctgt cgtgccagcg 2460

gatccgcatc tcaattagtc agcaaccata gtcccgcccc taactccgcc catcccgccc 2520

ctaactccgc ccagttccgc ccattctccg ccccatggct gactaatttt ttttatttat 2580

gcagaggccg aggccgcctc ggcctctgag ctattccaga agtagtgagg aggctttttt 2640

ggaggcctag gcttttgcaa aaagctaact tgtttattgc agcttataat ggttacaaat 2700

aaagcaatag catcacaaat ttcacaaata aagcattttt ttcactgcat tctagttgtg 2760

gtttgtccaa actcatcaat gtatcttatc atgtctggat ccgctgcatt aatgaatcgg 2820

ccaacgcgcg gggagaggcg gtttgcgtat tgggcgctct tccgcttcct cgctcactga 2880

ctcgctgcgc tcggtcgttc ggctgcggcg agcggtatca gctcactcaa aggcggtaat 2940

acggttatcc acagaatcag gggataacgc aggaaagaac atgtgagcaa aaggccagca 3000

aaaggccagg aaccgtaaaa aggccgcgtt gctggcgttt ttccataggc tccgcccccc 3060

tgacgagcat cacaaaaatc gacgctcaag tcagaggtgg cgaaacccga caggactata 3120

aagataccag gcgtttcccc ctggaagctc cctcgtgcgc tctcctgttc cgaccctgcc 3180

gcttaccgga tacctgtccg cctttctccc ttcgggaagc gtggcgcttt ctcaatgctc 3240

acgctgtagg tatctcagtt cggtgtaggt cgttcgctcc aagctgggct gtgtgcacga 3300

accccccgtt cagcccgacc gctgcgcctt atccggtaac tatcgtcttg agtccaaccc 3360

ggtaagacac gacttatcgc cactggcagc agccactggt aacaggatta gcagagcgag 3420

gtatgtaggc ggtgctacag agttcttgaa gtggtggcct aactacggct acactagaag 3480

gacagtattt ggtatctgcg ctctgctgaa gccagttacc ttcggaaaaa gagttggtag 3540

ctcttgatcc ggcaaacaaa ccaccgctgg tagcggtggt ttttttgttt gcaagcagca 3600

gattacgcgc agaaaaaaag gatctcaaga agatcctttg atcttttcta cggggtctga 3660

cgctcagtgg aacgaaaact cacgttaagg gattttggtc atgagattat caaaaaggat 3720

cttcacctag atccttttaa attaaaaatg aagttttaaa tcaatctaaa gtatatatga 3780

gtaaacttgg tctgacagtt accaatgctt aatcagtgag gcacctatct cagcgatctg 3840

tctatttcgt tcatccatag ttgcctgact ccccgtcgtg tagataacta cgatacggga 3900

gggcttacca tctggcccca gtgctgcaat gataccgcga gacccacgct caccggctcc 3960

agatttatca gcaataaacc agccagccgg aagggccgag cgcagaagtg gtcctgcaac 4020

tttatccgcc tccatccagt ctattaattg ttgccgggaa gctagagtaa gtagttcgcc 4080

agttaatagt ttgcgcaacg ttgttgccat tgctacaggc atcgtggtgt cacgctcgtc 4140

gtttggtatg gcttcattca gctccggttc ccaacgatca aggcgagtta catgatcccc 4200

catgttgtgc aaaaaagcgg ttagctcctt cggtcctccg atcgttgtca gaagtaagtt 4260

ggccgcagtg ttatcactca tggttatggc agcactgcat aattctctta ctgtcatgcc 4320

atccgtaaga tgcttttctg tgactggtga gtactcaacc aagtcattct gagaatagtg 4380

tatgcggcga ccgagttgct cttgcccggc gtcaatacgg gataataccg cgccacatag 4440

cagaacttta aaagtgctca tcattggaaa acgttcttcg gggcgaaaac tctcaaggat 4500

cttaccgctg ttgagatcca gttcgatgta acccactcgt gcacccaact gatcttcagc 4560

atcttttact ttcaccagcg tttctgggtg agcaaaaaca ggaaggcaaa atgccgcaaa 4620

aaagggaata agggcgacac ggaaatgttg aatactcata ctcttccttt ttcaatatta 4680

ttgaagcatt tatcagggtt attgtctcat gagcggatac atatttgaat gtatttagaa 4740

aaataaacaa ataggggttc cgcgcacatt tccccgaaaa gtgccacctg 4790

<210> 2
<211> 3648
<212> DNA
<213> Artificial Sequence

<220>

<223>  pHWX

<400>  2
cagctggttc tttccgcctc agaagccata gagcccaccg catccccagc atgcctgcta      60

ttgtcttccc aatcctcccc cttgctgtcc tgccccaccc caccccccag aatagaatga      120

cacctactca gacaatgcga tgcaatttcc tcattttatt aggaaaggac agtgggagtg      180

gcaccttcca gggtcaagga aggcacgggg gaggggcaaa caacagatgg ctggcaacta      240

gaaggcacag tcgaggctga tcagcgagct ctagcattta ggtgaccgcc gggagggcgt      300

ccccggcccg gcgctgctcc cgcgtgtgtc ctggggttga ccagagggcc ccgggcgctc      360

cgtgtgtggc tgcgatggtg gcgtttttgg ggacaggtgt ccgtgtcgcg cgtcgcctgg      420

gccggcggcg tggtcggtga cgcgacctcc cggccccggg ggaggtatat ctttcgctcc      480

gagtcggcat tttgggccgc cgggttattg gagacggggt cgacctgcag actggctgtg      540

tataagggag cctgacattt atattcccca gaacatcagg ttaatggcgt ttttgatgtc      600

attttcgcgg tggctgagat cagccacttc ttccccgata acggaaaccg gcacactggc      660

catatcggtg gtcatcatgc gccagctttc atccccgata tgcaccaccg ggtaaagttc      720

acgggagact ttatctgaca gcagacgtgc actggccagg gggatcacca tccgtcgccc      780

gggcgtgtca ataatatcac tctgtacatc cacaaacaga cgataacggc tctctctttt      840

ataggtgtaa accttaaact gcatttcacc agcccctgtt ctcgtcagca aaagagccgt      900

tcatttcaat aaaccgggcg acctcagcca tcccttcctg attttccgct ttccagcgtt      960

cggcacgcag acgacgggct tcattctgca tggttgtgct taccagaccg gagatattga      1020

catcatatat gccttgagca actgatagct gtcgctgtca actgtcactg taatacgctg      1080

cttcatagca tacctctttt tgacatactt cgggtataca tatcagtata tattcttata      1140

ccgcaaaaat cagcgcgcaa atacgcatac tgttatctgg cttttagtaa gccggatcca      1200

cgcgtcgtct cctccccccc aacttcggag gtcgaccagt actccggtta actgctagac      1260

cctatagtga gtcgtattaa tttcgataag ccagtaagca gtgggttctc tagttagcca      1320

gagagctctg cttatataga cctcccaccg tacacgccta ccgcccattt gcgtcaatgg      1380

ggcggagttg ttacgacatt ttggaaagtc ccgttgattt tggtgccaaa acaaactccc      1440

attgacgtca atggggtgga gacttggaaa tccccgtgag tcaaaccgct atccacgccc      1500

attgatgtac tgccaaaacc gcatcaccat ggtaatagcg atgactaata cgtagatgta      1560

ctgccaagta ggaaagtccc ataaggtcat gtactgggca taatgccagg cgggccattt      1620

accgtcattg acgtcaatag ggggcgtact tggcatatcg acgtcaggtg gcacttttcg      1680

gggaaatgtg cgcggaaccc ctatttgttt atttttctaa atacattcaa atatgtatcc      1740

gctcatgaga caataaccct gataaatgct tcaataatat tgaaaaagga agagtatgag      1800

tattcaacat ttccgtgtcg cccttattcc cttttttgcg gcattttgcc ttcctgtttt 1860

tgctcaccca gaaacgctgg tgaaagtaaa agatgctgaa gatcagttgg gtgcacgagt 1920

gggttacatc gaactggatc tcaacagcgg taagatcctt gagagttttc gccccgaaga 1980

acgttttcca atgatgagca cttttaaagt tctgctatgt ggcgcggtat tatcccgtat 2040

tgacgccggg caagagcaac tcggtcgccg catacactat tctcagaatg acttggttga 2100

gtactcacca gtcacagaaa agcatcttac ggatggcatg acagtaagag aattatgcag 2160

tgctgccata accatgagtg ataacactgc ggccaactta cttctgacaa cgatcggagg 2220

accgaaggag ctaaccgctt ttttgcacaa catgggggat catgtaactc gccttgatcg 2280

ttgggaaccg gagctgaatg aagccatacc aaacgacgag cgtgacacca cgatgcctgt 2340

agcaatggca acaacgttgc gcaaactatt aactggcgaa ctacttactc tagcttcccg 2400

gcaacaatta atagactgga tggaggcgga taaagttgca ggaccacttc tgcgctcggc 2460

ccttccggct ggctggttta ttgctgataa atctggagcc ggtgagcgtg gttctcgcgg 2520

tatcattgca gcactggggc cagatggtaa gccctcccgt atcgtagtta tctacacgac 2580

ggggagtcag gcaactatgg atgaacgaaa tagacagatc gctgagatag gtgcctcact 2640

gattaagcat tggtaactgt cagaccaagt ttactcatat atactttaga ttgatttaaa 2700

acttcatttt taatttaaaa ggatctaggt gaagatcctt tttgataatc tcatgaccaa 2760

aatcccttaa cgtgagtttt cgttccactg agcgtcagac cccgtagaaa agatcaaagg 2820

atcttcttga gatccttttt ttctgcgcgt aatctgctgc ttgcaaacaa aaaaaccacc 2880

gctaccagcg gtggtttgtt tgccggatca agagctacca actctttttc cgaaggtaac 2940

tggcttcagc agagcgcaga taccaaatac tgttcttcta gtgtagccgt agttaggcca 3000

ccacttcaag aactctgtag caccgcctac atacctcgct ctgctaatcc tgttaccagt 3060

ggctgctgcc agtggcgata agtcgtgtct taccgggttg gactcaagac gatagttacc 3120

ggataaggcg cagcggtcgg gctgaacggg gggttcgtgc acacagccca gcttggagcg 3180

aacgacctac accgaactga gatacctaca gcgtgagcta tgagaaagcg ccacgcttcc 3240

cgaagggaga aaggcggaca ggtatccggt aagcggcagg gtcggaacag gagagcgcac 3300

gagggagctt ccagggggaa acgcctggta tctttatagt cctgtcgggt ttcgccacct 3360

ctgacttgag cgtcgatttt tgtgatgctc gtcagggggg cggagcctat ggaaaaacgc 3420

cagcaacgcg gcctttttac ggttcctggc cttttgctgg ccttttgctc acatgttctt 3480

tcctgcgtta tcccctgatt ctgtggataa ccgtattacc gcctttgagt gagctgatac 3540

cgctcgccgc agccgaacga ccgagcgcag cgagtcagtg agcgaggaag cggaagagcg 3600

cccaatacgc aaaccgcctc tccccgcgcg ttggccgatt cattaatg 3648

<210> 3
<211> 2280
<212> DNA
<213> Influenza A virus

<400> 3
atggagagaa taaaagaact gagagatcta atgtcgcagt cccgcactcg cgagatactc 60

actaagacca ctgtggacca tatggccata atcaaaaagt acacatcagg aaggcaagag 120

aagaaccccg cactcagaat gaagtggatg atggcaatga gatacccaat tacagcagac 180

aagagaataa tggacatgat tccagagagg aatgaacaag gacaaaccct ctggagcaaa 240

acaaacgatg ctggatcaga ccgagtgatg gtatcacctc tggccgtaac atggtggaat 300

aggaatggcc caacaacaag tacagttcat taccctaagg tatataaaac ttatttcgaa 360

aaggtcgaaa ggttgaaaca tggtaccttc ggccctgtcc acttcagaaa tcaagttaaa 420

ataaggagga gagttgatac aaaccctggc catgcagatc tcagtgccaa ggaggcacag 480

gatgtgatta tggaagttgt tttcccaaat gaagtggggg caagaatact gacatcagag 540

tcacagctgg caataacaaa agagaagaaa gaagagctcc aggattgtaa aattgctccc 600

ttgatggtgg cgtacatgct agaaagagaa ttggtccgta aaacaaggtt tctcccagta 660

gccggcggaa caggcagtgt ttatattgaa gtgttgcact taacccaagg gacgtgctgg 720

gagcagatgt acactccagg aggagaagtg agaaatgatg atgttgacca aagtttgatt 780

atcgctgcta gaaacatagt aagaagagca gcagtgtcag cagacccatt agcatctctc 840

ttggaaatgt gccacagcac acagattgga ggagtaagga tggtggacat ccttagacag 900

aatccaactg aggaacaagc cgtagacata tgcaaggcag caatagggtt gaggattagc 960

tcatctttca gttttggtgg gttcactttc aaaaggacaa gcggatcatc agtcaagaaa 1020

gaagaagaag tgctaacggg caacctccaa acactgaaaa taagagtaca tgaagggtat 1080

gaagaattca caatggttgg gagaagagca acagctattc tcagaaaggc aaccaggaga 1140

ttgatccagt tgatagtaag cgggagagac gagcagtcaa ttgctgaggc aataattgtg 1200

gccatggtat tctcacagga ggattgcatg atcaaggcag ttaggggcga tctgaacttt 1260

gtcaataggg caaaccagcg actgaacccc atgcaccaac tcttgaggca tttccaaaaa 1320

gatgcaaaag tgcttttcca gaactgggga attgaatcca tcgacaatgt gatgggaatg 1380

atcggaatac tgcccgacat gaccccaagc acggagatgt cgctgagagg gataagagtc 1440

agcaaaatgg gagtagatga atactccagc acggagagag tggtagtgag tattgaccga 1500

tttttaaggg ttagagatca aagagggaac gtactattgt ctcccgaaga agtcagtgaa 1560

acgcaaggaa ctgagaagtt gacaataact tattcgtcat caatgatgtg ggagatcaat 1620

ggccctgagt cagtgctagt caacacttat caatggataa tcaggaactg ggaaattgtg 1680

aaaattcaat ggtcacaaga tcccacaatg ttatacaaca aaatggaatt tgaaccattt 1740

cagtctcttg tccctaaggc aaccagaagc cggtacagtg gattcgtaag gacactgttc 1800

cagcaaatgc gggatgtgct tgggacattt gacactgtcc aaataataaa acttctcccc 1860

tttgctgctg ccccaccaga acagagtagg atgcaatttt cctcattgac tgtgaatgtg 1920

agaggatcag ggttgaggat actggtaaga ggcaattctc cagtattcaa ttacaacaag 1980

gcaaccaaac gacttacagt tcttggaaag gatgcaggtg cattgactga agatccagat 2040

gaaggcacat ctggggtgga gtctgctgtc ctgagaggat ttctcatttt gggcaaagaa 2100

gacaagagat atggcccagc attaagcatc aatgaactga gcaatcttgc aaaaggagag 2160

aaggctaatg tgctaattgg gcaaggggac gtagtgttgg taatgaaacg aaaacgggac 2220

tctagcatac ttactgacag ccagacagcg accaaaagaa ttcggatggc catcaattag 2280

<210> 4
<211> 2341
<212> DNA
<213> Influenza A virus

<400> 4
agcaaaagca ggcaaaccat ttgaatggat gtcaatccga ctctactttt cctaaaaatt 60

ccagcgcaaa atgccataag caccacattc ccttatactg gagatcctcc atacagccat 120

ggaacaggaa caggatacac catggacaca gtaaacagaa cacaccaata ctcagaaaag 180

ggaaagtgga cgacaaacac agagactggt gcaccccagc tcaacccgat tgatggacca 240

ctacctgagg ataatgaacc aagtgggtat gcacaaacag actgtgttct agaggctatg 300

gctttccttg aagaatccca cccaggaata tttgagaatt catgccttga aacaatggaa 360

gttgttcaac aaacaagggt agataaacta actcaaggtc gccagactta tgattggaca 420

ttaaacagaa atcaaccggc agcaactgca ttggccaaca ccatagaagt ctttagatcg 480

aatggcctaa cagctaatga gtcaggaagg ctaatagatt tcttaaagga tgtaatggaa 540

tcaatgaaca aagaggaaat agagataaca acccactttc aaagaaaaag gagagtaaga 600

gacaacatga ccaagaagat ggtcacgcaa agaacaatag ggaagaaaaa acaaagactg 660

aataagagag gctatctaat aagagcactg acattaaata cgatgaccaa agatgcagag 720

agaggcaagt taaaaagaag ggctatcgca acacctggga tgcagattag aggtttcgta 780

tactttgttg aaactttagc taggagcatt tgcgaaaagc ttgaacagtc tgggctccca 840

gtagggggca atgaaaagaa ggccaaactg gcaaatgttg tgagaaagat gatgactaat 900

tcacaagaca cagagatttc tttcacaatc actggggaca acactaagtg gaatgaaaat 960

caaaatcctc gaatgttcct ggcgatgatt acatatatca ccagaaatca acccgagtgg 1020

ttcagaaaca tcctgagcat ggcacccata atgttctcaa acaaaatggc aagactaggg 1080

aaagggtaca tgttcgagag taaaagaatg aagattcgaa cacaaatacc agcagaaatg 1140

ctagcaagca ttgacctgaa gtacttcaat gaatcaacaa agaagaaaat tgagaaaata 1200

aggcctcttc taatagatgg cacagcatca ctgagtcctg ggatgatgat gggcatgttc 1260

aacatgctaa gtacggtctt gggagtctcg atactgaatc ttggacaaaa gaaatacacc 1320

aagacaatat actggtggga tgggctccaa tcatccgacg attttgctct catagtgaat 1380

gcaccaaacc atgagggaat acaagcagga gtggacagat tctacaggac ctgcaagtta 1440

gtgggaatca acatgagcaa aaagaagtcc tatataaata agacagggac atttgaattc 1500

acaagctttt tttatcgcta tggatttgtg gctaatttta gcatggagct acccagcttt 1560

ggagtgtctg gagtaaatga atcagctgac atgagtattg gagtaacagt gataaagaac 1620

aacatgataa acaatgacct tggacctgca acggcccaga tggctcttca attgttcatc 1680

aaagactaca gatacacata taggtgccat aggggagaca cacaaattca gacgagaaga 1740

tcatttgagt taaagaagct gtgggatcaa acccaatcaa aagtagggct attagtatca 1800

gatggaggac caaacttata caatatacgg aatcttcaca ttcctgaagt ctgcttaaaa 1860

tgggagctaa tggatgatga ttatcgggga agactttgta atcccctgaa tccctttgtc 1920

agtcataaag agattgattc tgtaaacaat gctgtggtaa tgccagccca tggtccagcc 1980

aaaagcatgg aatatgatgc cgttgcaact acacattcct ggattcccaa gaggaatcgt 2040

tctattctca acacaagcca aaggggaatt cttgaggatg aacagatgta ccagaagtgc 2100

tgcaatctat tcgagaaatt tttccctagc agttcatata ggagaccggt tggaatttct 2160

agcatggtgg aggccatggt gtctagggcc cggattgatg ccagggtcga cttcgagtct 2220

ggacggatca agaaagaaga gttctctgag atcatgaaga tctgttccac cattgaagaa 2280

ctcagacggc aaaaataatg aatttaactt gtccttcatg aaaaaatgcc ttgtttctac 2340

t 2341

<210> 5
<211> 2151
<212> DNA
<213> Influenza A virus

<400> 5
atggaagact ttgtgcgaca atgcttcaat ccaatgatcg tcgagcttgc ggaaaaggca 60

atgaaagaat atggggaaga tccgaaaatc gaaactaaca agtttgctgc aatatgcaca 120

catttggaag tttgtttcat gtattcggat ttccatttca tcgacgaacg gggtgaatca 180

ataattgtag aatctggtga cccgaatgca ctattgaagc accgatttga gataattgaa 240

ggaagagacc gaatcatggc ctggacagtg gtgaacagta tatgtaacac aacaggggta 300

gagaagccta aatttcttcc tgatttgtat gattacaaag agaaccggtt cattgaaatt 360

ggagtaacac ggagggaagt ccacatatat tacctagaga aagccaacaa aataaaatct 420

gagaagacac acattcacat cttttcattc actggagagg agatggccac caaagcggac 480

tacacccttg acgaagagag cagggcaaga atcaaaacta ggcttttcac tataagacaa 540

gaaatggcca gtaggagtct atgggattcc tttcgtcagt ccgaaagagg cgaagagaca 600

attgaagaaa aatttgagat tacaggaact atgcgcaagc ttgccgacca aagtctccca 660

ccgaacttcc ccagccttga aaactttaga gcctatgtag atggattcga gccgaacggc 720

tgcattgagg gcaagctttc ccaaatgtca aaagaagtga acgccaaaat tgaaccattc 780

ttgaggacga caccacgccc cctcagattg cctgatgggc ctctttgcca tcagcggtca 840

aagttcctgc tgatggatgc tctgaaatta agtattgaag acccgagtca cgagggggag 900

ggaataccac tatatgatgc aatcaaatgc atgaagacat tctttggctg gaaagagcct 960

aacatagtca aaccacatga gaaaggcata aatcccaatt acctcatggc ttggaagcag 1020

gtgctagcag agctacagga cattgaaaat gaagagaaga tcccaaggac aaagaacatg 1080

aagagaacaa gccaattgaa gtgggcactc ggtgaaaata tggcaccaga aaaagtagac 1140

tttgatgact gcaaagatgt tggagacctt aaacagtatg acagtgatga gccagagccc 1200

agatctctag caagctgggt ccaaaatgaa ttcaataagg catgtgaatt gactgattca 1260

agctggatag aacttgatga aataggagaa gatgttgccc cgattgaaca tatcgcaagc 1320

atgaggagga actattttac agcagaagtg tcccactgca gggctactga atacataatg 1380

aagggagtgt acataaatac ggccttgctc aatgcatcct gtgcagccat ggatgacttt 1440

cagctgatcc caatgataag caaatgtagg accaaagaag gaagacggaa aacaaacctg 1500

tatgggttca ttataaaagg aaggtctcat ttgagaaatg atactgatgt ggtgaacttt 1560

gtaagtatgg agttctcact cactgacccg agactggagc cacacaaatg ggaaaaatac 1620

tgtgttcttg aaataggaga catgctcttg aggactgcga taggccaagt gtcgaggccc 1680

atgttcctat atgtgagaac caatggaacc tccaagatca agatgaaatg gggcatggaa 1740

atgaggcgct gccttcttca gtctcttcag cagattgaga gcatgattga ggccgagtct 1800

tctgtcaaag agaaagacat gaccaaggaa ttctttgaaa acaaatcgga aacatggcca 1860

atcggagagt cacccagggg agtggaggaa ggctctattg ggaaagtgtg caggacctta 1920

ctggcaaaat ctgtattcaa cagtctatat gcgtctccac aacttgaggg gttttcggct 1980

gaatctagaa aattgcttct cattgttcag gcacttaggg acaacctgga acctggaacc 2040

ttcgatcttg gggggctata tgaagcaatc gaggagtgcc tgattaatga tccctgggtt 2100

ttgcttaatg catcttggtt caactccttc ctcacacatg cactgaagta g 2151

<210> 6
<211> 1497
<212> DNA

<213>  Influenza A virus

<400>  6
atggcgtctc aaggcaccaa acgatcatat gaacaaatgg agactggtgg ggagcgccag      60

gatgccacag aaatcagagc atctgtcgga agaatgattg gtggaatcgg gagattctac     120

atccaaatgt gcactgaact caaactcagt gattatgatg gacgactaat ccagaatagc     180

ataacaatag agaggatggt gctttctgct tttgatgaga gaagaaataa atacctagaa     240

gagcatccca gtgctgggaa ggaccctaag aaaacaggag gacccatata tagaagagta     300

gacggaaagt ggatgagaga actcatcctt tatgacaaag aagaaataag gagagtttgg     360

cgccaagcaa acaatggcga agatgcaaca gcaggtctta ctcatatcat gatttggcat     420

tccaacctga atgatgccac atatcagaga acaagagcgc ttgttcgcac cggaatggat     480

cccagaatgt gctctctaat gcaaggttca acacttccca gaaggtctgg tgccgcaggt     540

gctgcggtga aaggagttgg aacaatagca atggagttaa tcagaatgat caaacgtgga     600

atcaatgacc gaaatttctg gaggggtgaa aatggacgaa ggacaagggt tgcttatgaa     660

agaatgtgca atatcctcaa aggaaaattt caaacagctg cccagagggc aatgatggat     720

caagtaagag aaagtcgaaa cccaggaaac gctgagattg aagacctcat tttcctggca     780

cggtcagcac tcattctgag gggatcagtt gcacataaat cctgcctgcc tgcttgtgtg     840

tatgggcttg cagtagcaag tgggcatgac tttgaaaggg aagggtactc actggtcggg     900

atagacccat tcaaattact ccaaaacagc caagtggtca gcctgatgag accaaatgaa     960

aacccagctc acaagagtca attggtgtgg atggcatgcc actctgctgc atttgaagat    1020

ttaagagtat caagtttcat aagaggaaag aaagtgattc caagaggaaa gctttccaca    1080

agaggggtcc agattgcttc aaatgagaat gtggaaacca tggactccaa taccctggaa    1140

ctgagaagca gatactgggc cataaggacc aggagtggag gaaataccaa tcaacaaaag    1200

gcatccgcag gccagatcag tgtgcagcct acattctcag tgcagcggaa tctccctttt    1260

gaaagagcaa ccgttatggc agcattcagc gggaacaatg aaggacggac atccgacatg    1320

cgaacagaag ttataagaat gatggaaagt gcaaagccag aagatttgtc cttccagggg    1380

cggggagtct tcgagctctc ggacgaaaag gcaacgaacc cgatcgtgcc ttcctttgac    1440

atgagtaatg aagggtctta tttcttcgga gacaatgcag aggagtatga cagttga       1497


<210>  7
<211>  982
<212>  DNA
<213>  Influenza A virus

<400>  7
atgagtcttc taaccgaggt cgaaacgtac gttctttcta tcatcccgtc aggccccctc      60

aaagccgaga tcgcgcagag actggaaagt gtctttgcag gaaagaacac agatcttgag     120

gctctcatgg aatggctaaa gacaagacca atcttgtcac ctctgactaa gggaatttta 180

ggatttgtgt tcacgctcac cgtgcccagt gagcgaggac tgcagcgtag acgctttgtc 240

caaaatgccc taaatgggaa tggggacccg aacaacatgg atagagcagt taaactatac 300

aagaagctca aaagagaaat aacgttccat ggggccaagg aggtgtcact aagctattca 360

actggtgcac ttgccagttg catgggcctc atatacaaca ggatgggaac agtgaccaca 420

gaagctgctt ttggtctagt gtgtgccact tgtgaacaga ttgctgattc acagcatcgg 480

tctcacagac agatggctac taccaccaat ccactaatca ggcatgaaaa cagaatggtg 540

ctggctagca ctacggcaaa ggctatggaa cagatggctg gatcgagtga acaggcagcg 600

gaggccatgg aggttgctaa tcagactagg cagatggtac atgcaatgag aactattggg 660

actcatccta gctccagtgc tggtctgaaa gatgaccttc ttgaaaattt gcaggcctac 720

cagaagcgaa tgggagtgca gatgcagcga ttcaagtgat cctctcgtca ttgcagcaaa 780

tatcattggg atcttgcacc tgatattgtg gattactgat cgtctttttt tcaaatgtat 840

ttatcgtcgc tttaaatacg gtttgaaaag agggccttct acggaaggag tgcctgagtc 900

catgagggaa gaatatcaac aggaacagca gagtgctgtg gatgttgacg atggtcattt 960

tgtcaacata gagctagagt aa 982

<210> 8
<211> 1775
<212> DNA
<213> Influenza A virus

<400> 8
   agcaaaagca ggggaaaata aaaacaacca aaatgaaagc aaaactactg gtcctgttat 60
   gtacatttac agctacatat gcagacacaa tatgtatagg ctaccatgcc aacaactcaa 120
   ccgacactgt tgacacagta cttgagaaga atgtgacagt gacacactct gtcaacctac 180
   ttgaggacag tcacaatgga aaactatgtc tactaaaagg aatagcccca ctacaattgg 240
   gtaattgcag cgttgccgga tggatcttag gaaacccaga atgcgaatta ctgatttcca 300
   aggaatcatg gtcctacatt gtagaaacac caaatcctga gaatggaaca tgttacccag 360
   ggtatttcgc cgactatgag gaactgaggg agcaattgag ttcagtatct tcatttgaga 420
   gattcgaaat attccccaaa gaaagctcat ggcccaccca caccgtaacc ggagtatcag 480
   catcatgctc ccataatggg aaaagcagtt tttacagaaa tttgctatgg ctgacgggga 540
   agaatggttt gtacccaaac ctgagcaagt cctatgtaaa caacaaagag aaagaagtcc 600
   ttgtactatg gggtgttcat cacccgccta acatagggga ccaaaggccc ctctatcata 660
   cagaaaatgc ttatgtctct gtagtgtctt cacattatag cagaagattc accccagaaa 720
   tagccaaaag acccaaagta agagatcagg aaggaagaat caactactac tggactctgc 780
   tggaacctgg ggatacaata atatttgagg caaatggaaa tctaatagcg ccatggtatg 840
   cttttgcact gagtagaggc tttggatcag gaatcatcac ctcaaatgca ccaatggatg 900
   aatgtgatgc gaagtgtcaa acacctcagg gagctataaa cagcagtctt cctttccaga 960
   atgtacaccc agtcacaata ggagagtgtc caaagtatgt caggagtgca aaattaagga 1020
   tggttacagg actaaggaac atcccatcca ttcaatccag aggtttgttt ggagccattg 1080
   ccggtttcat tgaagggggg tggactggaa tggtagatgg gtggtatggt tatcatcatc 1140
   agaatgagca aggatctggc tatgctgcag atcaaaaaag tacacaaaat gccattaacg 1200
   ggattacaaa caaggtgaat tctgtaattg agaaaatgaa cactcaattc acagctgtgg 1260
   gcaaagaatt caacaaattg gaaagaagga tggaaaactt aaataaaaaa gttgatgatg 1320
   ggtttctaga catttggaca tataatgcag aattgttggt tctactggaa aatgaaagga 1380
   ctttggattt ccatgactcc aatgtgaaga atctgtatga gaaagtaaaa agccaattaa 1440
   agaataatgc caaagaaata ggaaacgggt gttttgaatt ctatcacaag tgtaacaatg 1500
   aatgcatgga gagtgtgaaa aatggaactt atgactatcc aaaatattcc gaagaatcaa 1560
   agttaaacag ggagaaaatt gatggagtga aattggaatc aatgggagtc tatcagattc 1620
   tggcgatcta ctcaactgtc gccagttccc tggttctttt ggtctccctg ggggcaatca 1680
   gcttctggat gtgttccaat gggtctttgc agtgtagaat atgcatctga gaccagaatt 1740
   tcagaaatat aagaaaaaac acccttgttt ctact 1775

<210> 9
<211> 1414
<212> DNA
<213> Influenza A virus

<400> 9
aatgaatcca aatcaaaaaa taataaccat tggatcaatc agtatagcaa tcggaataat 60

tagtctaatg ttgcaaatag gaaatattat ttcaatatgg gctagtcact caatccaaac 120

tggaagtcaa aaccacactg gagtatgcaa ccaaagaatc atcacatatg aaaacagcac 180

ctgggtgaat cacacatatg ttaatattaa caacactaat gttgttgctg gaaaggacaa 240

aacttcagtg acattggccg gcaattcatc tctttgttct atcagtggat gggctatata 300

cacaaaagac aacagcataa gaattggctc caaaggagat gtttttgtca taagagaacc 360

tttcatatca tgttctcact tggaatgcag aacctttttt ctgacccaag gtgctctatt 420

aaatgacaaa cattcaaatg ggaccgttaa ggacagaagt ccttataggg ccttaatgag 480

ctgtcctcta ggtgaagctc cgtccccata caattcaaag tttgaatcag ttgcatggtc 540

agcaagcgca tgccatgatg gcatgggctg gttaacaatc ggaatttctg gtccagacaa 600

tggagctgtg gctgtactaa aatacaacgg cataataact gaaaccataa aaagttggaa 660

aaagcgaata ttaagaacac aagagtctga atgtgtctgt gtgaacgggt catgtttcac 720

cataatgacc gatggcccga gtaatggggc cgcctcgtac aaaatcttca agatcgaaaa 780

ggggaaggtt actaaatcaa tagagttgaa tgcacccaat tttcattatg aggaatgttc 840

ctgttaccca gacactggca cagtgatgtg tgtatgcagg gacaactggc atggttcaaa 900

tcgaccttgg gtgtctttta atcaaaacct ggattatcaa ataggataca tctgcagtgg 960

ggtgttcggt gacaatccgc gtcccaaaga tggagagggc agctgtaatc cagtgactgt 1020

tgatggagca gacggagtaa aggggttttc atacaaatat ggtaatggtg tttggatagg 1080

aaggactaaa agtaacagac ttagaaaggg gtttgagatg atttgggatc ctaatggatg 1140

gacagatacc gacagtgatt tctcagtgaa acaggatgtt gtggcaataa ctgattggtc 1200

agggtacagc ggaagtttcg ttcaacatcc tgagttaaca ggattggact gtataagacc 1260

ttgcttctgg gttgagttag tcagaggact gcctagagaa aatacaacaa tctggactag 1320

tgggagcagc atttcttttt gtggcgtaaa tagtgatact gcaaactggt cttggccaga 1380

cggtgctgag ttgccgttca ccattgacaa gtag 1414